# EUROPEAN PATENT SPECIFICATION

(11) **EP 2 162 013 B1**
(45) Date of publication and mention of the grant of the patent: **06.04.2016**
(21) Application number: 07766611.3
(22) Date of filing: 06.07.2007
(51) Int. Cl.: A23G 4/08, A23G 4/18, A23G 4/20, A61K 9/00, A23L 5/00

(54) **COMPRESSED CHEWING GUM COMPRISING AN ENCAPSULATION DELIVERY SYSTEM COMPRISING NATURAL RESIN**
KOMPRIMIERTER KAUGUMMI MIT EINEM NATURHARZ UMFASSENDEN VERKAPSELUNGSDARREICHUNGSSYSTEM
GOMME À MÂCHER COMPRESSÉE COMPRENANT UN SYSTÈME DE DÉLIVRANCE PAR ENCAPSULATION COMPRENANT UNE RÉSINE NATURELLE

(43) Date of publication of application: 17.03.2010
(73) Proprietor: Gumlink A/S, 7100 Vejle (DK)
(72) Inventor: THORENGAARD, Bitten, DK-7120 Vejle Øst (DK)
(74) Representative: Patentgruppen
(86) International application number: PCT/IB2007/001898
(87) International publication number: WO 2009/007767

(56) References cited:
- EP-A- 0 401 954
- EP-A- 0 401 956
- WO-A-2006/127738
- WO-A-2007/140286

## Description

### FIELD OF THE INVENTION

The invention relates to a compressible chewing gum composition according to claim 1.

### BACKGROUND OF THE INVENTION

Compressed chewing gum is known as a chewing gum which is obtained by compression of a compressible chewing gum composition in a tabletting press.

The compressible chewing gum composition typically comprises chewing gum granules and may further comprise various chewing gum ingredients and active ingredients in powder form. The technique of gathering the chewing gum by compression provides unique possibilities of including various ingredients, even those which are vulnerable to the conventional process of mixing a chewing gum composition, which is afterwards formed into individual pieces, e.g. by rolling and scoring.

Ever since compressed chewing gum was invented, it has been a problem to control the release of ingredients. The compressed chewing gum tends to release all water soluble ingredients relatively immediate during the initial chewing phase.

The problem of too quick release from the compressed chewing gum has been dealt with in WO 2006/127738 which discloses compressed chewing gum comprising an encapsulation delivery system. Certain ingredients intended to be released more slowly are encapsulated in this encapsulation delivery system.

EP 0 401 956 discloses a chewing gum composition with sustained sweetener release through a high melting-point delivery system comprising a particulate sweetener and an encapsulating matrix comprising a blend of materials.

WO 2007/140286 disc1 oses an oral composition for stain removal from a tooth surface, wherein the composition comprises an encapsulated protease enzyme.

It is an object of the invention to obtain an improved encapsulation delivery system to obtain prolonged or alternative release profiles of compressed chewing gum.

### SUMMARY OF THE INVENTION

The present invention relates to a compressible chewing gum composition comprising i) chewing gum granules containing gum base and ii) particles of one or more encapsulation delivery systems comprising at least one encapsulation material and at least one active ingredient being encapsulated by the encapsulation material, wherein the encapsulation material comprises at least one natural resin,
wherein said encapsulation material further comprises polyvinyl acetate, and wherein there is a ratio of said natural resin to said polyvinyl acetate in the range of 95:5 to 5:95, preferably in the range of 70:30 to 15:85 such as in the range of 60:40 to 20:80.

The compressible chewing gum composition, which has been provided, is suitable for compression into compressed chewing gum tablets. Such compressed chewing gum tablets thus comprise a compression of a quantity of the compressible chewing gum composition and thus comprise chewing gum granules containing gum base and particles of one or more encapsulation delivery systems comprising active ingredient encapsulated by an encapsulation material comprising natural resin.

According to the invention, it has surprisingly been found that in compressed chewing gum, an encapsulation delivery system comprising natural resin as at least a part of the encapsulation material, which encapsulates one or more active ingredients, results in advantageous release profiles. The release of active ingredient from compressed chewing gum may be modified in advantageous ways by application of natural resin as at least a part of the encapsulation material, and the invention provides new possibilities of combining different encapsulation delivery systems in compressed chewing gum.

It is noted that the natural resin applied according to the provisions of the invention is a little less hydrophilic than e.g. PVA typically applied for encapsulation purposes in compressed chewing gum. This feature of the natural resin may have the function of delaying the release from the encapsulation system a little, thereby invoking a strongly desired prolonged release of he encapsulated ingredient when applied in compressed chewing gum.

In an embodiment of the invention, said at least one natural resin constitutes 15 % to 95 %, and preferably in the range of 25 % to 95 % by weight of said encapsulation material.

According to the invention, encapsulation of active ingredients in an encapsulation material comprising natural resin provides an encapsulation delivery system which is suitable in a compressible chewing gum composition. It has been found that such an encapsulation delivery system serves to delay the release of active ingredient significantly as compared to the release of un-encapsulated active ingredient.

It has moreover been found that it is actually possible to substitute at least a part of the polyvinyl acetate, which is widely used as encapsulation material within the art.

In an embodiment of the invention, at least one of said encapsulation delivery systems comprises a combination of two or more natural resins.

Advantageous embodiments of the invention may be provided, when two or more natural resins are combined in the same encapsulation delivery system. Mixtures of more than one natural resin may be applied as a factor in providing not only a prolonged release but also a controlled release.

In an embodiment of the invention, the compressible chewing gum composition comprises an amount of the one or more encapsulation delivery systems in the range of 0.5 % to 20 % by weight, preferably in the range of 1 % to 10 % by weight, and most preferably in the range of 2 % to 6 % by weight of the composition.

The amount of encapsulation delivery system in the compressible chewing gum composition and the resulting compressed chewing gum is dependent on the load of active ingredient in the encapsulation delivery system and on the desired content of active ingredient in the chewing gum. In case the active ingredient needs to be active ingredient in the chewing gum. In case the active ingredient needs to be metered very accurate, the percentage of encapsulation delivery system is balanced accordingly in the compressible chewing gum composition.

In an embodiment of the invention, the size of the particles of said one or more encapsulation delivery systems is in the range of 100 - 3000 µm, preferably in the range of 100 - 2000 µm, preferably 100-1000 such as in the range of 100 - 600 µm.

In an embodiment of the invention, the size of the particles of one or more of the encapsulation delivery systems is in the range of 100 - 1500 µm, such as in the range of 100 - 600 µm.

Preferably, the size of the particles of encapsulation delivery system is relatively small. In compressed chewing gum, too large particles will "fall out" of the compressed composition during chewing of the compressed chewing gum.

The particle size of the encapsulation delivery systems may affect both the release rate of active ingredient and the mouth-feel of the final compressed chewing gum. Encapsulation delivery system particles of relatively large size may be applied to obtain a more delayed release as compared to smaller particles of the same encapsulation delivery system.

The compressible chewing gum composition may comprise encapsulation delivery systems, which are particulated into different size distributions. The particle size of the encapsulation delivery system may in this way be applied as one of the parameters for controlling the release of active ingredient in a compressed chewing gum.

In an embodiment of the invention, said at least one natural resin is selected from the group consisting of polyterpene resins, hydrogenated resins, polymerized resins, or any combination thereof.

According to the invention, hydrogenated resins, polymerized resins and in particular polyterpene resins have been found to be very suitable as encapsulation materials for encapsulation of active ingredients in compressed chewing gum.

In an embodiment of the invention, said polyterpene resins are selected from the group consisting of polymerized monoterpenes, polymerized cyclic monoterpenes, polymerized delta-limonene, polymerized alpha-pinene, polymerized beta-pinene, styrenated polyterpene, or any combination thereof.

In an embodiment of the invention, said polyterpene resin has a softening point in the range of 70°C to 150°C, preferably in the range of 80°C to 120°C, and most preferably in the range of 80°C to 105°C.

In an embodiment of the invention, said polyterpene resin has a molecular weight (Mn) in the range of 400 - 4000 g/mol, preferably in the range of 500 - 2000 g/mol.

Even though the molecular weight of the preferred polyterpene resins is rather low compared to most polymeric encapsulation materials, it has been found that these polyterpene resins are in fact very effective as encapsulation materials and serves to prolong the period during which the active ingredient is released.

Without being bound to any theory, the effectiveness of the polyterpene resins as encapsulation material may be related to their relatively hydrophobic nature. The encapsulation of active ingredients within such hydrophobic material, as compared to a more hydrophilic material, may prolong the period of chewing during which the water in the saliva is gradually reaching all parts of the active ingredients within the encapsulation. Also prior to chewing, i.e. during storage of the chewing gum tablets, the hydrophobic nature of the polyterpene resin may have an advantageous effect, as it may serve to protect the encapsulated active ingredient from any moisture in the environment.

According to the invention, said encapsulation material further comprises polyvinyl acetate.

According to the invention, the encapsulation material may advantageously comprise a combination of natural resin and polyvinyl acetate. Although polyvinyl acetate may be regarded as a relatively hydrophilic polymer, it is has been found that it may be mixed with natural resin such as polyterpene resin to form a combined encapsulation material, which is very suitable in a compressible chewing gum composition according to the invention.

According to the invention, there is a ratio of said natural resin to said polyvinyl acetate in the range of 95:5 to 5:95, preferably in the range of 70:30 to 15:85.

According the invention, very advantageous release profiles of active ingredient from compressed chewing gum are obtained when the active ingredient is encapsulated in an encapsulation material comprising a mixture of polyvinyl acetate and natural resin, such as polyterpene resin.

Moreover, the advantage is obtained from the natural resin that the ability of the encapsulation material to protect the active ingredient is improved. As it has been found according to the invention that natural resin is in fact suitable for encapsulation and prolonging of release of active ingredient from compressed chewing gum, several further advantages may moreover be obtained. The natural resin, such as polyterpene resin, may provide a very effective protection of the active ingredient, and thus improve the storage ability of the particles of encapsulation delivery system and consequently of the compressible chewing gum composition. Thereby, the storage and transportation time may be extended without compromising the quality of the active ingredient. Also during the process of tabletting the compressible chewing gum composition to form the compressed tablets, the natural resin may provide an effective protection of vulnerable active ingredients. Furthermore, in the period of time between manufacturing and use of the final compressed chewing gum tablets with a content of encapsulated active ingredient, a high proportion of natural resin in the encapsulation material may have the effect that the quality of the active ingredient is preserved.

Moreover, an advantage, which may be obtained when applying natural resin in the encapsulaton delivery system, is an improved stability of the encapsulation delivery system and compressible chewing gum composition and thus in the resulting chewing gum.

A further advantage obtained according to the invention is that in some embodiments, natural resin may provide a stickiness to the compressed chewing gum, which is typically difficult to obtain in compressed chewing gum.

In an embodiment of the invention, there is a ratio of said natural resin to said polyvinyl acetate in the range of 60:40 to 20:80, preferably in the range of 50:50 to 25:75.

In an embodiment of the invention, said encapsulation material constitutes in the range of 55 % to 95 %, preferably 60 % to 90 %, and most preferably 65 % to 80 % by weight of said encapsulation delivery system.

The higher percentages of encapsulation material are applied according to the invention, when it is desired to encapsulate the active ingredients to a large degree, perhaps more or less entirely, for example in order to achieve an effective protection of the active ingredient, or to obtain a slower release of the active ingredient. Application of less high percentages of encapsulation material may provide encapsulation delivery systems comprising a higher concentration of active ingredient. Thereby, a smaller amount of encapsulation delivery system may be sufficient in order to provide a certain concentration of active ingredient in the compressible chewing gum composition and the resulting compressed chewing gum.

In an embodiment of the invention, said polyvinyl acetate has a molecular weight (Mw) in the range of 5,000 to 40,000 g/mole.

In an embodiment of the invention, said polyvinyl acetate has a molecular weight (Mw) in the range of 40,000 to 100,000 g/mole.

In an embodiment of the invention, the tensile strength of the encapsulation delivery system is within the range of 40 - 1400 MPa, preferably in the range of 50 - 1200 MPa.

According to an advatageous embodiment of the invention, the tensile strength should be relatively low, i.e. between 6500-200,000 psi in order render the encapsulation sufficiently weak and thereby enable that the encapsulation system ends of releasing the encapsulated ingredients. This tensile strength is obtained though the application of natural resin, which may provide the necessary non-elasticity.

In an embodiment of the invention, said encapsulation delivery system further comprises one or more softeners.

In various embodiments of the invention, softeners are advantageously added in the encapsulation delivery system to soften the natural resin and any polymeric material applied as encapsulation material. The most suitable softeners have been found to be softeners, which are rather mild in their softening action, such as waxes and oils.

In an embodiment of the invention, at least one of said softeners is selected from the group consisting of waxes, fats, oils, or any combination thereof.

In an embodiment of the invention, said encapsulation material further comprises one or more elastomer in an amount of at most 15 %, preferably at most 10 %, and most preferably at most 5 % by weight.

According to an embodiment of the invention, the content of elastomer is preferably kept low. The elastomer may affect the tensile strength undesirably.

In an embodiment of the invention, said encapsulation delivery system further comprises one or more anti-caking agents.

In an embodiment of the invention, two or more encapsulation delivery systems comprise two or more active ingredients.

Combining different encapsulaton delivery systems in one compressible chewing gum composition provides the possibility of controlling the release of two or more of active ingredients at the same time. The active ingredients may be of the same or of different sorts. Each encapsulation delivery system can be designed for a specific active ingredient, and the application of natural resin in one or more of the encapsulation delivery systems may provide attractive release profiles of certain active ingredients such as pharmaceutical active ingredients, food acids, and high intensity sweeteners.

In an advantageous embodiment of the invention, the application of natural resin in one or more encapsulation delivery systems may provide the possibility of obtaining an initial fast release and a continuously stable release, which is not reduced within a chewing period of at least 20 minutes, preferably at least 40 minutes.

In an embodiment of the invention, the one or more encapsulation delivery systems comprise at least a first encapsulation delivery system and at least a second encapsulation delivery system comprising different percentages of natural resin.

According to an advantageous embodiment of the invention, different release properties of the at least two encapsulation delivery systems may be combined to form a desired release profile of the final compressed chewing gum.

In an embodiment of the invention, the one or more encapsulation delivery systems comprise at least a first encapsulation delivery system and at least a second encapsulation delivery system comprising different encapsulation material.

In an embodiment of the invention, said compressible chewing gum composition comprises one or more further encapsulation delivery systems comprising polyvinyl acetate as encapsulation material.

According to a preferred embodiment of the invention, a controlled release may be obtained by having an active ingredient in an encapsulation delivery system comprising polyvinyl acetate as encapsulation material, while the same or another sort of active ingredient is comprised in the encapsulation delivery system comprising at least one natural resin, one of the encapsulating delivery system is a mixture of natural resin and polyvinyl acetate as encapsulation material.

In an embodiment of the invention, the one or more encapsulation delivery systems comprise at least a first encapsulation delivery system comprising at least a first active ingredient and at least a second encapsulation delivery system comprising at least a second active ingredient.

In an embodiment of the invention, the active ingredient is distributed in the encapsulation delivery system as particles with particle sizes in the range of 0.1-100 µm, preferably in the range of 1-50 µm.

In an embodiment of the invention, said active ingredient comprises at least one pharmaceutical active ingredient.

In an embodiment of the invention, said active ingredient comprises at least one nutraceutical active ingredient.

In an embodiment of the invention, said active ingredient is selected from the group consisting of high intensity sweeteners, food acids, flavors, cooling agents, warming agents, or any combination thereof.

In an embodiment of the invention, said active ingredient constitutes in the range of 1 % to 90 % by weight of the encapsulation delivery system.

Typically, the basic idea of the encapsulation delivery system is to provide particles with a high content of active ingredients to be encapsulated and subsequently released.

In an embodiment of the invention, said active ingredient constitutes in the range of 1 % to 80 % by weight of the encapsulation delivery system.

In an embodiment of the invention, said active ingredient constitutes in the range of 1 % to 70 % by weight of the encapsulation delivery system.

In an embodiment of the invention, said active ingredient constitutes in the range of 1 % to 50 % by weight of the encapsulation delivery system.

In an embodiment of the invention, said active ingredient constitutes in the range of 5 % to 45 % by weight of the encapsulation delivery system.

In an embodiment of the invention, said active ingredient constitutes in the range of 10 % to 45 % by weight of the encapsulation delivery system.

According to an advantageous embodiment of the invention, a relatively high amount of active ingredient may be applied in order to provide the desired effect by the relevant active ingredient by means of relatively "few" "high intensity" particles when compared to the number or amount of gum base granules.

In other words, the concentration of the relevant active ingredient should be different and higher in the particles of the encapsulation system if active ingredients are incorporated in the gum base granules.

In an embodiment of the invention, the content of active ingredient is different in said chewing gum granules and said particles of encapsulation delivery systems.

A further and particular advantage obtained according to the invention by applying active ingredients in particles of encapsulation delivery system instead of in the chewing gum granules is that interaction between chewing gum ingredients and active ingredients is avoided. Moreover, the separation of active ingredients from the composition of the chewing gum granules implies that the texture of the chewing gum granules may be adjusted without regard to preserving the active ingredients.

A further advantage according to the invention is obtained, as the one or more encapsulation delivery systems make it possible to control the release of active ingredient without affecting the formulation of the chewing gum granules and thus without compromising the texture properties of the chewing gum granules.

Moreover, the encapsulation delivery system may be manufactured by a careful process taking the precautions to avoid damage of vulnerable active ingredients. The mechanical or physical handling of the encapsulation delivery system may be performed with care as compared to the process of manufacturing of chewing gum granules. For example, as regards the temperature during manufacture, the temperature may be kept lower in order to avoid damage of the active ingredients.

In an embodiment of the invention, it is only necessary to adjust the encapsulation delivery system in order to obtain a desired release profile, and it is possible to use a standard gum base and chewing gum formulation in the compressible chewing gum composition and the compressed chewing gum.
In an embodiment of the invention, the chewing gum granules are free of active ingredient.

In an embodiment of the invention, said high intensity sweeteners are selected from the group consisting of aspartame, acesulfame-K or other salts of acesulfame, alitame, sucralose, neotame, NEPH, saccharin and its salts, cyclamic acid and its salts, glycyrrhizin, dihydrochalcones, thaumatin, monellin, stevioside, or any mixture thereof.

High intensity sweeteners may advantageously be applied for the purpose of enhancing the sweetening perception of other sweetening agents or flavors.

In an embodiment of the invention, said food acids are selected from the group consisting of citric acid, tartaric acid, malic acid, fumaric acid, succinic acid, ascorbic acid, adipic acid, lactic acid, or any mixture thereof.

According to an embodiment of the invention, the high intensity sweeteners and/or food acids may applied in ground form in particle sizes in the range of 0.1 - 100 µm, preferably in the range of 1 - 50 µm.

In an embodiment of the invention, said active ingredient is selected from the group consisting of cetirizine, nicotine, metformin, metformin HCl, phenylephrine, deca-peptide KSL-W, acesulfame K, aspartame, citric acid, malic acid, vitamin C, resveratrol, any variation thereof or any combination thereof.

In an embodiment of the invention, said active ingredient is selected from the group consisting of pharmaceuticals, nutraceuticals, medicaments, nutrients, nutritional supplements, drugs, dental care agents, herbals, and the like and combinations thereof.

In an embodiment of the invention, said active ingredient is selected from the ATC anatomical groups consisting of agents acting on:
**A** alimentary tract and metabolism, **B** blood and blood forming organs, **C** cardiovascular system, **D** dermatologicals, **G** genito urinary system and sex hormones, **H** systemic hormonal preparations, **J** antiinfectives for systemic use, **L** antineoplastic and immunomodulating agents, **M** musculo-skeletal system, **N** nervous system, **P** antiparasitic products, insecticides and repellents, **R** respiratory system and **S** sensory organs, **V** various, or any combination thereof.

In an embodiment of the invention, said active ingredient is selected from the therapeutical groups consisting of: Antipyretic, Anti allergic, Anti-arrytmic, Appetite suppressant, Anti-inflammatory, Broncho dilator, Cardiovascular drugs, Coronary dilator, Cerebral dilator, Peripheral vasodilator, Anti-infective, Psychotropic, Anti-manic, Stimulant, Decongestant, Gastro-intestinal sedative, Sexual dysfunction agent, Desinfectants, Anti-anginal substance, Vasodilator, Anti-hypertensive agent, Vasoconstrictor, Migraine treating agent, Anti-biotic, Tranquilizer, Anti-psychotic, Anti-tumor drug, Anticoagulant, Hypnotic, Sedative, Anti-emetic, Anti-nauseant, Anti-convulsant, Neuromuscular agent, Hyper and hypoglycaemic, Thyroid and anti-thyroid, Diuretic, Anti-spasmodic, Uterine relaxant, Anorectics, Spasmolytics, Anabolic agent, Erythropoietic agent, Anti-asthmatic, Expectorant, Cough suppressant, Mucolytic, Anti-uricemic agent, Dental vehicle, Breath freshener, Antacid, Anti-diuretic, Anti-flatulent, Betablocker, Teeth Whitener, Enzyme, Coenzyme, Protein, Energy Booster, Fiber, Probiotics, Prebiotics, Antimicrobial agent, NSAID, Anti-tussives, Decongestrants, Anti-histamines, Anti-diarrheals, Hydrogen antagonists, Proton pump inhibitors, General nonselective CNS depressants, General nonselective CNS stimulants, Selectively CNS function modifying drugs, Antiparkinsonism, Narcotic-analgetics, Analgetic-antipyretics, Psychopharmacological drugs, diagnostica sex hormones allergens, antifungal agents, Chronic Obstructive Pulmonary Disease (COPD) or any combination thereof.

In an embodiment of the invention, said active ingredient is selected from the group consisting of metformin, cetirizine, levo cetirizine, phenylephrine, flurbiprofen, nicotine, nicotine bitartrate, nicotine polacrilex, nicotine in combination with alkaline agents, nicotine in combination with caffeine, sodium picosulfate, fluor, fluor in combination with fruit acids, chlorhexidine, or any derivatives thereof, salts thereof, isomers thereof, nicotine antagonists, combinations thereof or compounds comprising one or more of these.

In an embodiment of the invention, said active ingredient is selected from the group consisting of: ace-inhibitors, antianginal drugs, antiarrhythrmas, anti-asthmatics, anti-cholesterolemics, analgesics, anesthetics, anticonvulsants, anti-depressants, antidiabetic agents, anti-diarrhea preparations, antidotes, anti-histamines, anti-hypertensive drugs, anti-inflammatory agents, anti-lipid agents, antimanics, anti-nauseants, anti-stroke agents, anti-thyroid preparations, anti-tumor drugs, anti-viral agents, acne drugs, alkaloids, amino acid preparations, anti-tussives, antiuricemic drugs, anti-viral drugs, anabolic preparations, systemic and non-systemic antiinfective agents, anti-neoplastics, anti-parkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, .endometriosis management agents, enzymes, erectile dysfunction therapies such as sildenafil citrate, which is currently marketed as Viagra™, fertility agents, gastrointestinal agents, homeopathic remedies, hormones, hypercalcemia and hypocalcemia management agents, immunomodulators, inmosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocryptine or nicotine, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anti-coagulants, antithrombotic drugs, hypnotics, anti-emetics, anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

In an embodiment of the invention, said active ingredient is selected from the group consisting of ephedrine, pseudo ephedrine, caffeine, loratadine, sildenafil, simvastatin, sumatriptan, acetaminophen, calcium carbonate, vitamin D, ibuprofen, aspirin, alginic acid in combination with aluminum hydroxide and sodium bicarbonate, ondansetron, Tibolon, Rimonabant, Varenicline, allergenes, sitagliptin or any derivatives thereof, salts thereof, isomers thereof, combinations thereof or compounds comprising one or more of these.

In an embodiment of the invention, said active ingredient is selected from the group consisting of phytochemicals, such as resveratrol and anthocyanine; herbals, such as green tea or thyme; antioxidants, such as polyphenols; micronutrients; mouth moisteners, such as acids; throat soothing ingredients; appetite suppressors; breath fresheners, such as zinc compounds or copper compounds; diet supplements; cold suppressors; cough suppressors; vitamins, such as vitamin A, vitamin C or vitamin E; minerals, such as chromium; metal ions; alkaline materials, such as carbonates; salts; herbals, dental care agents, such as remineralisation agents, antibacterial agents, anticaries agents, plaque acid buffering agents, tooth whiteners, stain removers or desensitizing agents; and combinations thereof.

In an embodiment of the invention, said active ingredient is selected from the group consisting of di-peptides, tri-peptides, oligo-peptides, deca-peptides, deca-peptide KSL, deca-peptide KSL-W, amino acids, proteins, or any combination thereof.

In an embodiment of the invention, said active ingredient comprises a probiotic bacteria, such as lactobacilli, bifidobacteria, lactococcus, streptococcus, leuconostoccus, pediococcus or enterococcus.

In an embodiment of the invention, said active ingredient comprises a prebiotic, such as fructose, galactose, mannose, insulin or soy.

In an embodiment of the invention, said chewing gum granules comprise gum base in an amount in the range of 90 % to 100 % by weight.

Advantageous embodiments of the invention may be obtained, when the chewing gum granules of the compressible chewing gum composition are merely constituted of gum base.

The granules or particles may be produced in several different ways. A gum base-containing granule or a particle may typically be produced substantially into the desired shape and size by means of an extrusion process or alternatively be produced on the basis of a gum base-containing mass which is subsequently separated into particles of a smaller size.

When dealing with non-gum base-containing granules or particles, these particles or granules may be produced as indicated above or e.g. by an agglomeration process of very small particles into the desired shape and size.

In an embodiment of the invention, said compressible chewing gum composition further comprises chewing gum additives selected from the group consisting of bulk sweeteners, high intensity sweeteners, flavors, fillers, colouring agents, softeners, emulsifiers, acidulants, antioxidants, cooling agents, warming agents, food acids, or any combination thereof.

Chewing gum ingredients such as bulk sweeteners may be included in the composition either as a part of the chewing gum granules or blended in particulate form with the composition of chewing gum granules and encapsulation delivery system particles, or both.

In an embodiment of the invention, the compressible chewing gum composition comprises further particles selected from the group consisting of bulk sweeteners, flavors, high intensity sweeteners, food acids, fillers, or any combination thereof.

In an embodiment of the invention, the chewing gum granules have average particle sizes within the range of 200 - 5000 µm, preferably in the range of 700 - 2500 µm.

In an embodiment of the invention, the gum base contained in the chewing gum granules comprises in the range of 20 % to 90 % by weight of the chewing gum granules.

In an embodiment of the invention, the chewing gum base comprises biodegradable polymers.

According to an embodiment of the invention, the gum base may be formed from biodegradable polymers and optionally further gum base ingredients such as fillers and softeners.

Moreover, the invention relates to compressed chewing gum tablet comprising a compressible chewing gum composition according to any of the claims 1-55.

Moreover, the invention relates to a compressed chewing gum tablet comprising
i) chewing gum granules containing gum base
ii) particles of one or more encapsulation delivery systems comprising at least one encapsulation material and at least one active ingredient being encapsulated by the encapsulation material,
wherein the encapsulation material comprises at least one natural resin and a polyvinyl acetate.

In an embodiment of the invention, the chewing gum consists of one module, such as a one-layered tablet

In an embodiment of the invention, the chewing gum comprises two or more modules and wherein at least two of the modules vary in composition.

In an embodiment of the invention, the tablet comprises at least two modules and wherein at least one of said modules comprises a chewing gum tablet module comprising at least one active ingredient and less than 5% gum base by weight of the module.

In an embodiment of the invention, the content of gum base is at least 6% by weight of the tablet.

In an embodiment of the invention, the content of gum base is at least 8% by weight of the tablet

In an embodiment of the invention, the content of gum base is at least 10% by weight of the tablet.

In an embodiment of the invention, the chewing gum tablet comprises chewing gum ingredients including sweetener, flavour, softener, and optionally filler.

In an embodiment of the invention, at least one encapsulation delivery system releases 80-100 % by weight of its active ingredient within the first 6 minutes of chewing the compressed chewing gum.

In an embodiment of the invention, at least one encapsulation delivery system releases 60-80 % by weight of its active ingredient within the first 6 minutes of chewing the compressed chewing gum.

In an embodiment of the invention, at least one encapsulation delivery system releases 30-60 % by weight of its active ingredient within the first 6 minutes of chewing the compressed chewing gum.

In an embodiment of the invention, said chewing gum tablet comprises one, two, three, four, five, six, or seven layers, at least one of which being composed of a compressible chewing gum composition according to any of the claims 1-55.

The layers may appear as tablet slice-like layers. The tabletting process for preparing the compressed chewing gum tablet with layers may involve feeding the material e.g. the compressible chewing gum composition for the first layer into a tabletting machine and performing a light compression of the first layer followed by feeding the material e.g. the compressible chewing gum composition for the second layer and performing a light compression, etc. until the desired number of layers is reached, and a final compression step is performed.

At least one layer of the layered compressed chewing gum tablet is composed of a compressible chewing gum composition according to the invention. However, one or more of the layers may be free of gum base and/or free of encapsulation delivery system. Such layers may for example be composed of bulk sweeteners, flavors, and additives.

In an embodiment of the invention, said chewing gum tablet comprises at least one layer of said compressible chewing gum composition and further comprises one, two, three, four, five, six, or seven layers of gum base free tablet material.

As used herein, the term "tablet material" refers to a material, which is substantially free of gum base. Typically, as used herein the tablet material mainly comprises bulk sweeteners and other chewing gum ingredients in powder form.

In an embodiment of the invention, said gum base free tablet material comprises bulk sweeteners.

In an embodiment of the invention, at least one of said layers of gum base free tablet material comprises one or more active ingredients

In an embodiment of the invention, the amount of water being below 2% by weight of the compressed tablet prior to compression.

In an embodiment of the invention, the amount of water being below 2% by weight of the compressed tablet after compression.

In an embodiment of the invention, the tablet is enclosed by an exterior protective barrier.

According to an advantageous embodiment of the invention, the protective barrier should form a humidity barrier.

In an embodiment of the invention, the exterior protective barrier is a blister pack, can, etc.

In an embodiment of the invention, the amount of water being below 2% by weight of the compressed tablet after further storage.

In an embodiment of the invention, the amount of water being below 2% by weight of the compressed tablet after 180 days of storage.

In an embodiment of the invention, at least one layer of said chewing gum tablet comprises a center-filling.

In an embodiment of the invention, the chewing gum tablet comprises
i) a first module of compressed chewing gum granules containing gum base,
ii) a second module of compressed material, and
iii) a centre filling of liquid, semi-liquid or solid material, said filling being a separate compartment located between said first and second layer and said filling being fully encapsulated within said compressed chewing gum tablet.

In an embodiment of the invention, the compressed tablet is in a form selected from the group consisting of a pellet, a cushion-shaped pellet, a stick, a tablet, a chunk, a pastille, a pill, a ball and a sphere, a figure, a box-shaped product, an edged product, a rounded product or any combination thereof.

In an embodiment of the invention, the compressed chewing gum tablet is coated by a coating selected from the group consisting of hard coatings, soft coatings, film coatings, or any combination thereof.

In an embodiment of the invention, said tablet comprises at least one polyol sweetener, at least one active ingredient and compressed particles of chewing gum base material, wherein the content of polyol is between 21 and 95% by weight of the tablet, and wherein the tablet being compressed at an ambient temperature of below 25 degrees Celsius and a maximum relative humidity of 55.

In an embodiment of the invention, said tablet comprises particles of chewing gum base material, wherein the content of gum base is at least 5% by weight of the tablet and wherein the chewing gum tablet is compressed at a pressure of more than 12 kN/cm² and wherein the chewing gum tablet has a volume above 0.15 cm³.

Furthermore, the invention relates to a method of providing a compressed chewing gum tablet from the compressible chewing gum composition according to any of the claims 1-55 comprising the step of compressing the compressible chewing gum composition in a tabletting press.

In an embodiment of the invention, the chewing gum tablet is compressed by application of a maximum force in the range of 20 to 80 kN in the tabletting press.

In an embodiment of the invention, the chewing gum tablet is compressed at a surrounding temperature of below 20°C and a maximum relative humidity of 55 %.

In an embodiment of the invention, the chewing gum granules are produced by means of cutting during an extrusion process.

In an embodiment of the invention, the chewing gum granules are produced by means of particulating a cooled chewing gum or gum base material.

Moreover, the invention relates to use of the compressible chewing gum composition according to any of the claims 1-55 to provide a compressed chewing gum.

### THE FIGURES

The invention will be described with reference to the figures where
- fig. 1: is a Scanning Electron Microscopy picture of particles of an encapsulation delivery system,
- fig. 2: is a schematic illustration of a cross-section of one particle of an encapsulation delivery system,
- fig. 3: illustrates results obtained from a taste panel blind testing the sweetness intensity of compressed chewing gum tablets during a chewing period, the tablets comprising aspartame located in different ways in the compressible chewing gum composition, either as free powder or within an encapsulation delivery system or as a part of the chewing gum granules,
- fig. 4: illustrates % release of aspartame from compressed chewing gum tablets by in vivo chew out studies and measurement by HPLC, comparing encapsulation in PVA with encapsulation in PVA:polyterpene resin (in a ratio of 3:1),
- fig. 5: illustrates results obtained from a taste panel blind testing the sweetness intensity of compressed aspartame containing chewing gum tablets during a chewing period,
- fig. 6: illustrates % release of acesulfame-K from compressed chewing gum tablets by in vivo chew out studies and measurement by HPLC, comparing encapsulation in PVA with encapsulation in PVA:polyterpene resin (in a ratio of 1:1),
- fig. 7: illustrates % release of aspartame from compressed chewing gum tablets by in vivo chew out studies and measurement by HPLC, encapsulation in PVA:polyterpene resin (in a ratio of 3:1) and comparing particle sizes of 106-500 µm and 500-1000 µm.

### DETAILED DESCRIPTION

In the following the compressible chewing gum composition and compressed chewing gum according to the invention are described in more details.
The compressible chewing gum composition according to the invention is applied in the manufacturing of compressed chewing gum tablets according to the invention. Conventional methods of compression of may be applied, for example such as the suitable methods disclosed in WO/2006/127738, WO/2006/079343, WO/2006/002622, and WO/2005/063038, hereby incorporated by reference.

The chewing gum granules in the compressible chewing gum composition according to the invention comprise at least a gum base portion, and provide generally the masticatory substance mainly imparting the chew characteristics to the final compressed chewing gum product.

In the present context, the terms chewing gum granule and chewing gum particle are used interchangeably with respect to size and shape in the sense that a granule or particle for use in a compression process is regarded to be a relatively small object, which together with other granules or particles may be compressed into a stable chewing gum tablet. The granules or particles may be produced in several different ways. A gum base-containing granule of particle may typically be produced substantially into the desired shape or size by means of an extrusion process or alternatively be produced on the basis of a gum base-containing mass which is subsequently separated into particles of a smaller size.

The gum base portion comprises water-insoluble ingredients which are retained in the mouth throughout the chewing period.

Conventionally, the gum base portion may merely define the release profile of flavours and sweeteners in the gum product. However, the gum base is also significant in defining the texture of the product, and thus the gum base polymers should be chosen to obtain the desired texture properties.

According to the present invention, encapsulation delivery systems, defined here below, are provided and applied in the compressible chewing gum composition and final compressed chewing gum product to control the release of active ingredients including high intensity sweeteners, flavouring agents, pharmaceutical active ingredients, nutraceuticals, food acids, etc.

It has been found that advantageous release profiles of active ingredients from compressed chewing gum may be obtained by encapsulation of the active ingredients in an encapsulation material comprising natural resin and a polyvinyl acetate. The encapsulation material and the active ingredient, which is encapsulated by the encapsulation material, are herein referred to in total as an encapsulation delivery system. Particles of the encapsulation delivery system form part of the compressible chewing gum composition according to the invention.

As used in this context, a particle of an encapsulation delivery system is a particle produced from a mixture or mass composed at least of encapsulation material and small active ingredient particles distributed therein in such way that they are encapsulated by the encapsulation material. The particles of encapsulation delivery system material may be produced by separating this mixture or mass into particles. The particles may be produced into a desired shape and size in a batch process or by means of an extrusion process.

Usually, the content of active ingredient in a particle of an encapsulation delivery system is higher than at least 5% by weight of the particle and higher than optionally incorporated active ingredients in the chewing gum granules.

Usually, the particles of encapsulation delivery system has a lower number of components than the number of components in the chewing gum granules.

The compressible chewing gum composition moreover comprises a water-soluble bulk portion and flavouring agents. The water-soluble bulk portion of which the major part comprises bulk sweetener dissipates with a portion of the flavouring agents over a period of time during chewing of the compressed chewing gum product. Various locations of the water soluble bulk portion in the final compressed chewing gum product are applicable. The water soluble bulk portion may e.g. be in particulate form and blended with the chewing gum granules and particles of encapsulation delivery system in the compressible chewing gum composition. Moreover, the water soluble bulk portion may e.g. be partly or entirely comprised in the chewing gum granules, and furthermore, the water soluble bulk portion may be partly or entirely located in a separate layer of the final compressed chewing gum product.

Turning to the formulation of the insoluble gum base portion of the chewing gum granules, the gum base may typically contain a combination of elastomers, vinyl polymers, elastomer plasticizers, waxes, softeners, fillers and other optional ingredients such as colourants and antioxidants.

The composition of gum base formulations can vary substantially depending on the particular product to be prepared and on the desired masticatory and other sensory characteristics of the final product. However, typical ranges (% by weight) of the above gum base components are: 5 to 50% by weight elastomeric compounds, 5 to 55% by weight elastomer plasticizers, 0 to 40% by weight of waxes, 5 to 35% by weight softener, 0 to 50% by weight filler, and 0 to 5% by weight of miscellaneous ingredients such as antioxidants, colourants, etc.

Elastomers provide the rubbery, cohesive nature to the gum, which varies depending on this ingredient's chemical structure and how it may be compounded with other ingredients. Elastomers suitable for use in the gum base and gum of the present invention may include natural or synthetic types. The elastomer may be any water-insoluble polymer known in the art, and includes those gum polymers utilized for chewing gum and bubble gum listed in Food and Drug Administration, CFR, Title 21, Section 172,615, as "Masticatory Substances of Natural Vegetable Origin" and "Masticatory Substances, Synthetic". Useful natural elastomers include natural rubber such as smoked or liquid latex and guayule, natural gums such as jelutong, lechi caspi, perillo, sorva, massaranduba balata, massaranduba chocolate, nispero, rosidinha, chicle, gutta percha, gutta kataiu, niger gutta, tunu, chilte, chiquibul, gutta hang kang. Useful synthetic elastomers include high molecular weight elastomers such as butadiene-styrene copolymers, polyisobutadiene and isobutylene-isoprene copolymers, low molecular weight elastomers such as polybutene, polybutadiene and polyisobutylene, vinyl polymeric elastomers such as polyvinyl acetate and polyethylene, vinyl copolymeric elastomers such as vinyl acetate/vinyl laurate, vinyl acetate/vinyl stearate, ethylene/vinyl acetate, polyvinyl alcohol or mixtures thereof.

Butadiene-styrene type elastomers, or SBR as they may be called, typically are copolymers of from about 20:80 to 60:40 styrenes:butadiene monomers. The ratio of these monomers affects the elasticity of the SBR as evaluated by mooney viscosity. As the styrene:butadiene ratio decreases, the mooney viscosity decreases.
The structure of SBR typically consists of straight chain 1,3-butadiene copolymerized with phenylethylene (styrene) and provides the non-linear molecular nature of these elastomers. The average molecular weight of SBR is <600.000 g/mole. Isobutylene-isoprene type elastomers, or butyl as they may be called, have molar percent levels of isoprene ranging from 0.2 to 4.0. Similar to SBR, as the isoprene:isobutylene ratio decreases, so does the elasticity, measured by mooney viscosity. The structure of butyl rubber typically consists of branched 2-methyl-1,3-butadiene (isoprene) copolymerized with branched 2-methylpropene (isobutylene), and, as with SBR, this type of structure is non-linear in nature. The average molecular weight of SBR is in the range from 150,000 g/mole to 1,000,000 g/mole. Polyisobutylene, or PIB as they may be called, type elastomers are polymers of 2-methylpropene and, as with SBR and butyl, are non-linear in nature. The low molecular weight elastomers provide soft chewing characteristics to the gum base and still provide the elastic qualities as do the other elastomers. Average molecular weights may range from about 30,000 to 120,000 g/mole and the penetration may range from about 4 millimetres to 20 millimetres. The higher the penetration, the softer the PIB. Similar to the SBR and butyl, the high molecular weight elastomers provide elasticity the gum. Average molecular weight may range from 120,000 to 1,000,000 g/mole. Polybutenes range in average molecular weight from about 5,000 g/mole to about 30,000 g/mole.

Vinyl polymeric and copolymeric type elastomers provide tack resistance, vary the chew characteristics of gums made from these bases having vinyl polymers and offer hydrophilic properties beneficial to sensory perception of the final gums. For vinyl copolymeric types, the amount of vinyl laurate, vinyl stearate, or ethylene present in the vinyl laurate/vinyl acetate (VL/VA), vinyl stearate/vinyl acetate (VS/VA), or ethylene/vinyl acetate (EVA) copolymers respectively typically ranges from about 10 to about 60% by weight of the copolymer. Average molecular weights of these polymers may range from about 2,000 g/mole to about 100,000 g/mole. The vinyl polymers such as polyvinyl alcohol and polyvinyl acetate have an average molecular weight from about 8,000 g/mole to about 65,000 g/mole.
Polymers of vinyl acetate (PVAc) are branched in nature. The degree of branching is increased when vinyl acetate monomers are copolymerized with vinyl laurate, vinyl stearate, ethylene and the like. The higher the degree of branching, the higher the compatibility when blended or compounded with normal-alkanic and iso-alkanic type waxes.

It is e.g. common in the industry to combine in a gum base a synthetic elastomer having a high molecular weight and a low-molecular-weight elastomer. Presently preferred combinations of synthetic elastomers include, but are not limited to, polyisobutylene and styrene-butadiene, polyisobutylene and polyisoprene, polyisobutylene and isobutylene-isoprene copolymer (butyl rubber) and a combination of polyisobutylene, styrene-butadiene copolymer and isobutylene isoprene copolymer, and all of the above individual synthetic polymers in admixture with polyvinyl acetate, vinyl acetate-vinyl laurate copolymers, respectively and mixtures thereof.

Elastomer plasticizers vary the firmness of the gum base. Their specificity on elastomer inter-molecular chain breaking (plasticizing) along with their varying softening points cause varying degrees of finished gum firmness and compatibility when used in base. This may be important when one wants to provide more elastomeric chain exposure to the alkanic chains of the waxes.

Elastomer plasticizers suitable for use in the present invention include natural rosin esters often referred to as ester gums. Such elastomer plasticizers known in the art are methyl, glycerol and pentaerythritol esters of rosins and modified rosins, such as hydrogenated, dimerized and polymerized rosins. Examples are glycerol ester of wood and gum rosin, glycerol ester of partially hydrogenated wood and gum rosin, glycerol ester of polymerized wood and gum rosin, glycerol ester of partially dimerized wood and gum rosin, glycerol ester of tall oil rosin, pentaerythritol ester of wood and gum rosin, pentaerythritol esters of partially and fully hydrogenated wood and gum rosin, methyl esters of wood and gum rosins and partially and fully hydrogenated methyl esters of wood and gum rosin.

The synthetic elastomer plasticizers include terpene resins derived from alpha-pinene, beta-pinene and/or d-limonene. The elastomer plasticizers used may be of one type or of combinations of more than one type. Typically, the ratios of one to the other are dependent on each respective softening point, the effect on flavour release, and the respective degree of tack they case to the gum. Ball and ring softening points of the rosin ester types described above may range from about 45°C. to about 120.degree. C. Softening points of the terpene resins may range from about 60.degree. C. to about 130.degree. C.

Petroleum waxes aid in the curing of the finished gum made from the gum base as well as improve shelf life and texture. Wax crystal size influences the release of flavour. Those waxes high in iso-alkanes have a smaller crystal size than those waxes high in normal-alkanes, especially those with normal-alkanes of carbon numbers less than 30. The smaller crystal size allows slower release of flavour since there is more hindrance of the flavour's escape from this wax versus a wax having larger crystal sizes. The compatibility of gum bases made using normal-alkanic waxes is less when compared to gum bases made with iso-alkanic waxes. Petroleum wax (refined paraffin and microcrystalline wax) and paraffin wax are composed of mainly straight-chained normal-alkanes and branched iso-alkanes. The ratio of normal-alkanes to iso-alkanes varies. The normal-alkanic waxes typically have carbon chain lengths >C-18 but the lengths are not predominantly longer than C-30. The branched and ring structures are located near the end of the chain for those waxes that are predominantly normal-alkanic. The viscosity of normal-alkanic waxes is <10 mm2/s (at 100 °C) and the combined number average molecular weight is <600 g/mole. The iso-alkanic waxes typically have carbon lengths that are predominantly greater than C-30. The branched chains and ring structures are located randomly along the carbon chain in those waxes that are predominantly iso-alkanic. The viscosity of iso-alkanic waxes is greater than 10 mm2/s (at 100 °C) and the combined number average molecular weight is >600 g/mole. Synthetic waxes are produced by means that are atypical for petroleum wax production and are thus not considered petroleum wax. The synthetic waxes may include waxes containing branched alkanes and copolymerized with monomers such as, but not limited to, propylene, polyethylene, and Fischer Tropsch type waxes. Polyethylene wax is a synthetic wax containing alkane units of varying lengths having attached thereto ethylene monomers. The natural waxes may include rice bran wax, bee's wax, carnauba wax or candelilla wax. The waxes may be used alone or in any combination.

The selection of softeners has an influence on the softness of the gum base. Softeners modify the texture, cause the hydrophobic and hydrophilic components of the base to be miscible, and may further plasticize the synthetic elastomers of the gum base. The emulsifiers, which belong to the group of softeners, provide the gum base with water-binding properties, which confer to the gum base a pleasant smooth surface and reduce its adhesive properties. Softeners suitable for use in the gum base include triglycerides of non-hydrogenated, partially hydrogenated and fully hydrogenated vegetable oils and tallow, cocoa butter and degreased cocoa powder and in addition to these the emulsifiers. The group of triglycerides includes cottonseed, palm, palm kernel, coconut, safflower, rapeseed, sunflower, tallow, soybean, cocoa butter, medium-chained triglycerides and the like. The caproic, caprylic, capric, myristic, lauric and palmitic fatty acids of the triglycerides tend to plasticize the synthetic elastomers more than triglycerides containing predominantly stearic fatty acid.

To the group of emulsifiers belong the monoglycerides, diglycerides, acetylated mono and diglycerides, distilled mono- and diglycerides, glycerol monostearate, propylene glycol monostearate, Na-, K-, Mg- and Ca-stearates, glycerol triacetate, fatty acid monoglycerides (e.g. stearic, palmitic, oleic and linoleic acids), lactic acid esters and acetic acid esters of mono- and diglycerides, sugar esters of edible fatty acids also referred to as sucrose polyesters including those disclosed in WO 00/25598, lecithin and hydroxylated lecithin. Most of these may contain triglyceride levels less than 2% by weight from their manufacturing processing.

The softeners including the emulsifiers may be used alone or at least two or more in combination.

Fillers used in gum base modify the texture of the gum base and aid in processing. Particle size has an effect on cohesiveness, density and processing characteristics of the gum base and its compounding. The smaller the particle size, the more dense and cohesive the final gum base. Also, by selecting fillers based on their particle size distribution, initial mass compounding may be varied, thus allowing alteration of the compounding characteristics of the initial mass during gum base processing and ultimately the final chew characteristics of gums made from these gum bases. Fillers suitable for use in the gum base include magnesium and calcium carbonate, ground limestone and silicate types such as magnesium and aluminum silicate, kaolin and clay, aluminium oxide, silicium oxide, talc, as well as titanium oxide, mono-, di-and tricalcium phosphate, sodium sulphate, cellulose polymers such as ethyl, methyl and wood or mixtures thereof.
Talc filler may be used in the gum base and gum of the present invention that may come in contact with or employ acid flavours or provide an acidic environment needed to prevent degradation of an artificial sweetener by reacting with calcium carbonate type fillers. Mean particle size for calcium carbonate and talc fillers typically range from about 0.1 micron to about 15 microns.
The fillers may also include natural organic fibres such as fruit vegetable fibres, grain, rice, cellulose and combinations thereof.

Antioxidants prolong shelf life and storage of gum base, finished gum or their respective components including fats and flavour oils. Antioxidants suitable for use in gum base include butylated hydroxyanisole (BHA), butylated hydroxytoluene (BHT), betacarotenes, tocopherols, acidulants such as Vitamin C, propyl gallate, other synthetic and natural types or mixtures thereof.

Flavourants and colourants impart characteristics or remove or mask undesired characteristics. Colourants may typically include FD&C type lakes, plant extracts, fruit and vegetable extracts and titanium dioxide flavourants may typically include cocoa powder, heat-modified amino acids and other vegetable extracts.

If desired, conventional elastomers or resins may be supplemented or substituted by biodegradable polymers.

Biodegradable polymers that may be used in the chewing gum of the present invention may be homopolymers, copolymers or terpolymers, including graft and block polymers.

Useful biodegradable polymers which may be applied as gum base polymers in the chewing gum of the present invention may generally be prepared by step-growth polymerization of di-, tri- or higher-functional alcohols or esters thereof with di-, tri-or higher-functional aliphatic or aromatic carboxylic acids or esters thereof. Likewise, also hydroxy acids or anhydrides and halides of polyfunctional carboxylic acids may be used as monomers. The polymerization may involve direct polyesterification or transesterification and may be catalyzed.

The usually preferred polyfunctional alcohols contain 2 to 100 carbon atoms as for instance polyglycols and polyglycerols.

Gum base polymers may both be resinous and elastomeric polymers.

In the polymerization of a gum base polymer for use in the chewing gum of the present invention, some applicable examples of alcohols, which may be employed as such or as derivatives thereof, include polyols such as ethylene glycol, 1,2-propanediol, 1,3-propanediol, 1,3-butanediol, 1,4-butanediol, 1,6-hexanediol, diethylene glycol, 1,4-cyclohexanediol, 1,4-cyclohexanedimethanol, neopentyl glycol, glycerol, trimethylolpropane, pentaerythritol, sorbitol, mannitol, etc.

Suitable examples of environmentally or biologically degradable chewing gum base polymers, which may be applied in accordance with the gum base of the present invention, include degradable polyesters, polycarbonates, polyester amides, polyesterurethanes, polyamides, prolamine, polypeptides, homopolymers of amino acids such as polylysine, and proteins including derivatives hereof such as e.g. protein hydrolysates including a zein hydrolysate.
Polyesters which may be applied in accordance with the gum base of the present invention may e.g. be as seen in EP 1 545 234 or EP 0 711 506 as incorporated herein by reference.

Further polymers which may be used in the gum base according to embodiments of the invention comprise:
enzymatically hydrolyzed zein, plasticized poly(D,L-lactic acid) and poly(D,L-lactic acid-co-glycolic acid), polyhydroxyalkanoates having side chain lengths of C₄ to C₃₀, poly(lactic acid) copolymers selected from the group consisting of poly(lactic acid-dimer fatty acid-oxazoline) copolymers and poly(lactic acid-diol-urethane) copolymers,
at least one polyester wherein the polyester includes monomers selected from the group consisting of lactic acid, lactide, glycolic acid, glycolide, citric acid, adipic acid, caprolactone, ethylene oxide, ethylene glycol, propylene oxide, and propylene glycol, and combinations thereof,
at least one polyester that is produced through a reaction of glycerol and at least one acid chosen from the group consisting of citric acid, fumaric acid, adipic acid, malic acid, succinic acid, suberic acid, sebacic acid, dodecanedioic acid, glucaric acid, glutamic acid, glutaric acid, azelaic acid, and tartaric acid,
at least one polyester that is produced through a reaction of at least one alcohol chosen from the group consisting of glycerol, propylene glycol, and 1,3 butylene diol, and at least one acid chosen from the group consisting of fumaric acid, adipic acid, malic acid, succinic acid, and tartaric acid, the polyester being end-capped with
a monofunctional ingredient selected from the group consisting of alcohols, acids, chlorides, and esters,
and further such as can be found in e.g. US 6,773,730, US 6,613,363, US 6,194,008, US 5,580,590, US 6,858,238, US 6,017,566, US 6,013,287, and US 5,800,848, which are all hereby incorporated by reference.

The polyesters formed on the basis of di- or polyfunctional acids and di- or polyfunctional alcohols may be produced according to known methods, one of which includes US2007/043200, hereby incorporated by reference.

The prolamine may e.g. be selected from the group consisting of zein, corn gluten meal, wheat gluten, gliadin, glutenin and any combination thereof. Methods of providing such a polymer are disclosed in US2004/001903, hereby incorporated by reference.

Examples of such protein-based compounds include but are not limited to prolamine, zein, corn gluten meal, wheat gluten, gliadin, glutenin and combinations thereof.

Such suitable biodegradable gum base polymers include polyesters, polycarbonates, polyesteramides, polyesterurethanes, polyamides, prolamine, and combinations thereof.

Polycarbonates may typically be co-polymerised with polyesters. Some typically preferred cyclic carbonates, which may be used as starting material, may e.g. comprise trimethylene carbonate, 2,2-dimethyltrimethylene carbonate, 2-methyltrimethylene carbonate, 3-methyltrimethylene carbonate, 2,3-dimethyltrimethylene carbonate, 2,4-dimethyltrimethylene carbonate, 2,3,4-trimethyltrimethylene carbonate, 2,3,3,4-tetramethyltrimethylene carbonate, etc.

In some embodiments, suitable polyesteramides can be constructed from monomers of the following groups: dialcohols, such as ethylene glycol, 1,4-butanediol, 1,3-propanediol, 1,6-hexanediol diethylene glycol and others; and/or dicarboxylic acid, such as oxalic acid, succinic acid, adipic acid and others, including those in the form of their respective esters (methyl, ethyl, etc.); and/or hydroxycarboxylic acids and lactones, such as caprolactone and others; and/or amino alcohols, such as ethanolamine, propanolamine, etc.; and/or cyclic lactams, such as .epsilon.-caprolactam or laurolactam, etc.; and/or .omega.-aminocarboxylic acids, such as aminocaproic acid, etc.; and/or mixtures (1:1 salts) of dicarboxylic acids such as adipic acid, succinic acid etc., and diamines such as hexamethyl enediamine, diaminobutane, etc.

In the case where the polymer mixture is based extensively on thermoplastic starch and an aromatic polyester, an aliphatic-aromatic copolyester, or a polyesteramide, it may be advantageous to add an aliphatic polyester or copolyester, such as polycaprolactone, for example, as a further component. As an example of this there may be mentioned a polymer mixture consisting of thermoplastic starch, at least one polyethylene terephthalate (PET) or a polyalkylene terephthalate, and polycaprolactone. Other examples of aliphatic polyesters or copolyesters are polylactic acid, polyhydroxybutyric acid, polyhydroxybutyric acid-hydroxyvaleric acid copolymer, and/or mixtures thereof.

Suitable polyesters may be obtained through polycondensation polymerisation or ring-opening polymerisation reactions. Some preferred polyesters include those polymerised from at least one carboxylic acid and at least one aliphatic di- or polyfunctional alcohols. The carboxylic acids may include aromatic dicarboxylic acids and aliphatic di- or polyfuncional carboxylic acids. In some preferred embodiments, the majority of the carboxylic acids are aliphatic.

Some of the preferred polyesters according to the invention may e.g. be prepared by step-growth polymerization of di-, tri- or higher-functional alcohols or esters thereof with di-, tri- or higher-functional aliphatic or aromatic carboxylic acids or esters thereof. Likewise, also hydroxy acids or anhydrides and halides of polyfunctional carboxylic acids may be used as monomers. The polymerization may involve direct polyesterification or transesterification and may be catalyzed. Use of branched monomers suppresses the crystallinity of the polyester polymers. Mixing of dissimilar monomer units along the chain also suppresses crystallinity. To control the reaction and the molecular weight of the resulting polymer it is possible to stop the polymer chains by addition of monofunctional alcohols or acids and/or to utilize a stoichiometric imbalance between acid groups and alcohol groups or derivatives of either. Also the adding of long chain aliphatic carboxylic acids or aromatic monocarboxylic acids may be used to control the degree of branching in the polymer and conversely multifunctional monomers are sometimes used to create branching. Moreover, following the polymerization monofunctional compounds may be used to end cap the free hydroxyl and carboxyl groups.

Examples of aliphatic di- or polyfunctional carboxylic acids, which may be applied as monomers of suitable polyesters include oxalic, malonic, citric, succinic, malic, tartaric, fumaric, maleic, glutaric, glutamic, adipic, glucaric, pimelic, suberic, azelaic, sebacic, dodecanedioic acid, etc. Likewise, specific examples of aromatic polyfunctional carboxylic acids may be terephthalic, isophthalic, phthalic, trimellitic, pyromellitic and naphthalene 1,4-, 2,3-, 2,6-dicarboxylic acids and the like. Some preferred polyesters are disclosed in CA2523510, hereby included by reference.

In a preferred embodiment, aliphatic dicarboxylic acids applied in the polyesters are selected from aliphatic dicarboxylic acids having from 4 to 12 carbons, such as succinic acid, glutaric acid, 2-methylglutaric acid, 3-methylglutaric acid, 2,2-dimethylglutaric acid, adipic acid, pimelic acid, suberic acid, azelaic acid and sebacic acid, higher homologues and stereoisomers and mixtures thereof. Preferred aliphatic dicarboxylic acids in this embodiment are succinic acid, glutaric acid, adipic acid, pimelic acid, azelaic acid and sebacic acid, and mixtures thereof.

In an embodiment, aromatic dicarboxylic acids applied in the polyesters contain two carboxyl groups which are bound to one aromatic system. Preferably, the aromatic system is carboaromatic, such as phenyl or naphthyl. In the case of polynuclear aromatics, the two carboxyl groups may be bound to the same ring or different rings. The aromatic system can also have one or more alkyl groups, for example methyl groups. The aromatic dicarboxylic acid is then generally selected from aromatic dicarboxylic acids having from 8 to 12 carbons, such as phthalic acid, isophthalic acid, terephthalic acid, 1,5- and 2,6-naphthalenedicarboxylic acid. Preferred aromatic dicarboxylic acids in this embodiment are terephthalic acid, isophthalic acid and phthalic acid and mixtures thereof.

Furthermore, some usually preferred polyfunctional alcohols suitable for preparing advantageous polyesters according to the invention contain 2 to 100 carbon atoms as for instance polyglycols and polyglycerols. Suitable examples of alcohols which may be employed in the polymerization process as such or as derivatives thereof include polyols such as ethylene glycol, 1,2-propanediol, 1,3-propanediol, 1,3-butanediol, 1,4-butanediol, 1,6-hexanediol, diethylene glycol, 1,4-cyclohexanediol, 1,4-cyclohexanedimethanol, neopentyl glycol, glycerol, trimethylolpropane, pentaerythritol, sorbitol, mannitol, etc. For the purpose of illustration and not limitation, some examples of alcohol derivatives include triacetin, glycerol palmitate, glycerol sebacate, glycerol adipate, tripropionin, etc.

Additionally, with regard to polyesters polymerized from alcohols or derivatives thereof and carboxylic acids or derivatives thereof, chain-stoppers sometimes used are monofunctional compounds. They are preferably either monohydroxy alcohols containing 1-20 carbon atoms or monocarboxylic acids containing 2-26 carbon atoms. General examples are medium or long-chain fatty alcohols or acids, and specific examples include monohydroxy alcohols such as methanol, ethanol, butanol, hexanol, octanol, etc., and lauryl alcohol, myristyl alcohol, cetyl alcohol, stearyl alcohol, stearic alcohol, etc., and monocarboxylic acids such as acetic, lauric, myristic, palmitic, stearic, arachidic, cerotic, dodecylenic, palmitoleic, oleic, linoleic, linolenic, erucic, benzoic, naphthoic acids and substituted napthoic acids, 1-methyl-2 naphthoic acid and 2-isopropyl-1-naphthoic acid, etc.

Typically, an acid catalyst or a transesterification catalyst may be used in such polyester polymerization processes, and non-limiting examples of those are the metal catalysts such as acetates of manganese, zinc, calcium, cobalt or magnesium, and antimony(III)oxide, germanium oxide or halide and tetraalkoxygermanium, titanium alkoxide, zinc or aluminum salts.
In a preferred embodiment of the invention, the polyesters can for example include copolymers containing any combination of the monomers lactic acid, lactide, glycolic acid, glycolide, citric acid, adipic acid, caprolactone, ethylene oxide, ethylene glycol, propylene oxide, propylene glycol and combinations thereof.

Examples of suitable polyesters obtainable by ring-opening polymerization include polyesters comprising combinations of cyclic monomers including the following:
D,L-lactide/ε-caprolactone,
   D,L-lactide/TMC
   D,L-lactide/δ-valerolactone
   D,L-lactide/dioxanone
   D,L-lactide
L-lactide/ε-caprolactone
   L-lactide/TMC
   L-lactide/δ-valerolactone
   L-lactide/dioxanone
   L-lactide
D,L-lactide/glycolide/ε-caprolactone
   D,L-lactide/glycolide/TMC
   D,L-lactide/glycolide/δ-valerolactone
   D,L-lactide/glylolide/dioxanone
   D,L-lactide/glycolide
L-lactide/glycolide/ε-caprolactone
   L-lactide/glycolide/TMC
   L-lactide/glycolide/δ-valerolactone
   L-lactide/glycolide/dioxanone
   L-lactide/glycolide
glycolide/ε-caprolactone
   glycolide/TMC
   glycolide/δ-valerolactone
   glycolide/dioxanone
   glycolide
D,L-lactide/L-lactide/ε-caprolactone
   D,L-lactide/L-lactide/TMC
   D,L-lactide/L-lactide/δ-valerolactone
   D,L-lactide/L-lactide/dioxanone
   D,L-lactide/L-lactide
D,L-lactide/L-lactide/glycolide/ε-caprolactone
   D,L-lactide/L-lactide/glycolide/TMC
   D,L-lactide/L-lactide/glycolide/δ-valerolactone
   D,L-lactide/L-lactide/glycolide/dioxanone
   D,L-lactide/L-lactide/glycolide

Some examples of the resulting polyester gum base polymers include poly (L-lactide-co-trimethylenecarbonate); poly (L-lactide-co-epsilon-caprolactone); poly (D, L-lactide-co-trimethylenecarbonate); poly (D, L-lactide-co-epsilon-caprolactone); poly (meso-lactide-co-trimethylenecarbonate); poly (mesolactide-co-epsilon-caprolactone); poly (glycolide-cotrimethylenecarbonate); poly (glycolide-co-epsilon-caprolactone), etc. Suitable polyesters are also disclosed in WO 2004/028270, hereby incorporated by reference.

In an embodiment, the polyesters may be obtained by the reaction between at least one dimer acid and at least one glycol or alcohol. Such glycols can include, for example, glycerin, propylene glycol, ethylene glycol, poly(ethylene glycol), poly(propylene glycol), poly(ethylene glycol-co-propylene glycol), while such alcohols can include, for example, methanol, ethanol, propanol, and butanol, and such dimer acids can include, for example, adipic acid and citric acid, etc.

Some specific examples of suitable polyesters include poly(lactic acid), polylactide, poly(glycolic acid), polyglycolide, poly(citric acid), polycaprolactone, polyhydroxyalkanoate, and combinations thereof.

Some suitable prolamines include zein, corn gluten meal, wheat gluten, gliadin, glutenin and combinations thereof. Moreover, blends of prolamine with polyester such as those disclosed in US 6,858,238, hereby included by reference, may be useful in chewing gum according to the invention.

In the water-soluble portion of the chewing gum, the ingredients may comprise softeners, sweeteners, high intensity sweeteners, flavouring agents, acidulants, fillers, antioxidants, and other components that provide desired attributes. Softeners typically constitute from about 0.5 percent to about 25.0 percent by weight of the chewing gum. The bulking agents generally comprise from about 5 percent to about 90 percent, preferably from about 20 percent to about 80 percent of the chewing gum. High-intensity sweeteners in gum typically may range from about 0.01 to 0.50 weight percent. A flavouring agent may be present in the chewing gum in an amount within the range of from about 0.1 to about 30.0 weight percent of the gum.

The softeners are added to the chewing gum in order to optimize the chewability and mouth feel of the gum.

Softeners contemplated for use in the gum include glycerin, modified lecithin and combinations thereof. Further aqueous sweetener solutions such as those containing sorbitol, hydrogenated starch hydrolysates, corn syrup and combinations thereof may be used as softeners.

The bulk sweeteners include both sugar and sugarless components. Bulk sweeteners may typically constitute 5 to about 95% by weight of the chewing gum, more typically constitute 20 to about 80% by weight, and, more commonly, 30 to 60% by weight of the gum. The sweeteners often fill the role of bulking agents in the gum. The sweeteners are improving juiciness of the gum and are supporting the flavour profile of the gum.

Sugar sweeteners generally include, but are not limited to, saccharide-containing components commonly known in the chewing gum art, such as sucrose, dextrose, maltose, saccharose, lactose, sorbose, dextrin, trehalose, D-tagatose, dried invert sugar, fructose, levulose, galactose, corn syrup solids, glucose syrup, hydrogenated glucose syrup, and the like, alone or in combination. The sugar sweeteners can be used in combination with sugarless sweeteners.

Sugarless sweeteners may also be applied as an alternative to sugar sweeteners to provide sugar-free chewing gum. Generally, sugarless sweeteners include components with sweetening characteristics but which are devoid of the commonly known sugars and comprise, but are not limited to, sugar alcohols such as sorbitol, mannitol, xylitol, hydrogenated starch hydrolyzates, maltitol, isomalt, erythritol, lactitol and the like, alone or in combination.

Depending on the particular sweetness release profile and shelf-life stability needed, bulk sweeteners can also be used in combination high-intensity sweeteners. Preferred high intensity sweeteners include, but are not limited to sucralose, aspartame, salts of acesulfame, alitame, saccharin and its salts, cyclamic acid and its salts, cyclamate, glycyrrhizin, dihydrochalcones, thaumatin, monellin, sterioside and the like, alone or in combination. In order to provide longer-lasting sweetness and flavour perception, it may be desirable to encapsulate or otherwise control the release of at least a portion of the artificial sweetener. Such techniques as wet granulation, wax granulation, spray drying, spray chilling, fluid bed coating, coascervation, encapsulation in yeast cells and fibre extrusion may be used to achieve the desired release characteristics. According to the invention, an advantageous encapsulation of high intensity sweeteners is obtained by the encapsulation delivery systems in which natural resins form part of the encapsulation material.

Usage level of the artificial sweetener will vary greatly and will depend on such factors as potency of the sweetener, rate of release, desired sweetness of the product, level and type of flavour used and cost considerations. Thus, the active level of artificial sweetener may vary from 0.02 to about 8% of the final chewing gum. When carriers used for encapsulation are included, the usage level of the encapsulated sweetener will be proportionally higher.

Additionally, the softener may also provide additional sweetness, if such softeners as aqueous sugar or alditol are used. If a low calorie gum is desired, a low caloric bulking agent can be used. Examples of low caloric bulking agents include polydextrose; Raftilose, Raftilin; Fructooligosaccharides (NutraFlora@); Palatinose oligosaccharide; Guar Gum Hydrolysate (SunFiber@); or indigestible dextrin (Fibersol). However, other low calorie-bulking agents can be used.

The chewing gum tablets provided herein may contain aroma agents and flavouring agents including natural and synthetic flavourings e.g. in the form of natural vegetable components, essential oils, essences, extracts, powders, including acids and other substances capable of affecting the taste profile. Examples of liquid and powdered flavourings include coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits (e.g. lemon, bergamot and orange) as mentioned above.

The chewing gum flavour may be a natural flavouring agent, which is freeze-dried, preferably in the form of a powder, slices or pieces of combinations thereof. The particle size may be less than 3 mm, preferably less than 2 mm, more preferably less than 1 mm, calculated as the longest dimension of the particle. The natural flavouring agent may be in a form where the particle size is from about 3 µm to 2 mm, such as from 4 µm to 1 mm. Preferred natural flavouring agents include seeds from a fruit e.g. from strawberry, blackberry and raspberry.

Various synthetic flavours, such as mixed fruit flavours may also be used in the present chewing gum centres. The aroma agent may be used in quantities smaller than those conventionally used. The aroma agents and/or flavours may be used in an amount of from 0.01 to about 30% by weight (preferably from 0.01 to about 15% by weight) of the final product depending on the desired intensity of the aroma and/or flavour used. Preferably, the content of aroma/flavour is in the range from 0.2 to 3% by weight of the total composition.

Also various acids are used typically in combination with fruit flavours, such as adipinic acid, succinic acid, fumaric acid, citric acid, tartaric acid, malic acid, acetic acid, lactic acid, phosphoric acid and glutaric acid or salts thereof. They are typically added in amounts of 0.01 to 10% by weight of the compressible chewing gum composition.

In the context of the present invention, the term "food acid" pertains to acids that are safe to use in food products. The food acids are typically mono-, di-, or tri-carboxylic acids. The food acids may e.g. be selected from the group consisting of citric acid, tartaric acid, malic acid, fumaric acid, succinic acid, ascorbic acid, adipic acid and lactic acid, and mixtures thereof. Phosphoric acid may also be a food acid according to the present invention.

Food acids suitable for encapsulation in an encapsulation delivery system as disclosed herein will typically be in a particulate form and may for example comprise a ground food acid.

Fillers suitable for use in the chewing gum include magnesium and calcium carbonate, ground limestone and silicate types such as magnesium and aluminum silicate, kaolin and clay, aluminium oxide, silicium oxide, talc, as well as titanium oxide, mono-, di- and tricalcium phosphate, sodium sulphate, cellulose polymers such as ethyl, methyl and wood or mixtures thereof.

Talc filler may be used in the chewing gum of the present invention that may come in contact with or employ acid flavours or provide an acidic environment needed to prevent degradation of an artificial sweetener by reacting with calcium carbonate type fillers. Mean particle size for calcium carbonate and talc fillers typically range from about 0.1 µm to about 15 µm.

The fillers may also include natural organic fibres such as fruit vegetable fibres, grain, rice, cellulose and combinations thereof.

Antioxidants prolong shelf life and storage of gum base, finished gum or their respective components including fats and flavour oils. Antioxidants suitable for use in gum base include butylated hydroxyanisole (BHA), butylated hydroxytoluene (BHT), betacarotenes, tocopherols, acidulants such as Vitamin C, propyl gallate, other synthetic and natural types or mixtures thereof.

Colourants and whiteners applied in the chewing gum according to the invention may include FD & C-type dyes and lakes, fruit and vegetable extracts, titanium dioxide, and combinations thereof.

Taste masking agents may be applied to improve the organoleptic properties of the product. The masking agents include sucralose, zinc gluconate, ethyl maltol, glycine, acesulfame-K, aspartame, saccharin, fructose, xylitol, spray dried licorice root, glycerrhizine, dextrose, sodium gluconate, glucono delta-lactone, ethyl vanillin, vanillin, normal and high-potency sweeteners, and a variety of appropriate flavors.

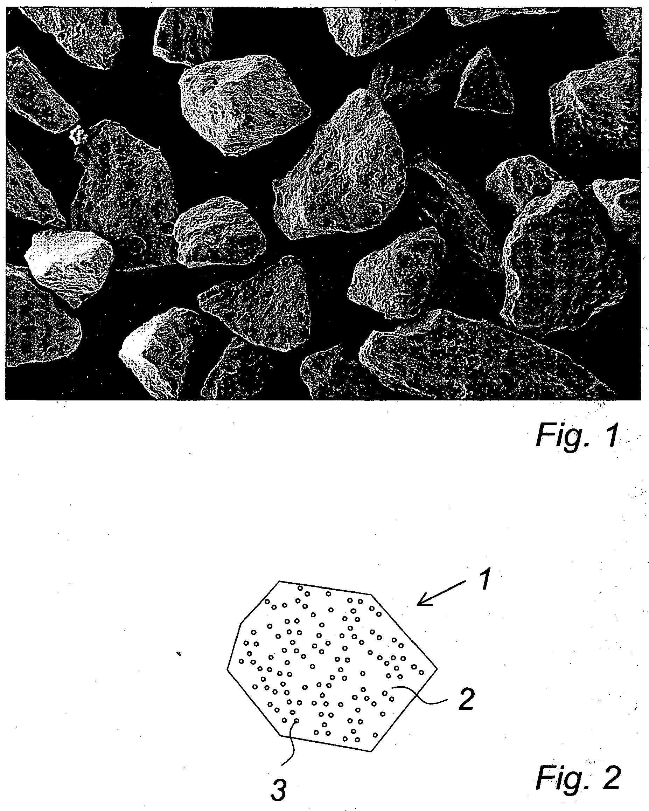

Fig. 1 shows in a Scanning Electron Microscopy picture an example of particles of an encapsulation delivery system.
Fig. 2 shows in a schematic illustration an example of a cross-section of a single particle 1 of an encapsulation delivery system. Small particles of active ingredient 3 are distributed in a matrix 2 composed of an encapsulation material as the main part and typically one or more softeners, and sometimes further components such as a detackifier. The encapsulation material contains at least one natural resin and may moreover contain e.g. synthetic polymeric materials such as polyvinyl acetate.

Typically, an encapsulation delivery system may be described as a particulate system, i.e. a system of particles each comprising the encapsulation material encapsulating the at least one active ingredient and typically comprising one or more softeners which soften the encapsulation material. As the softener is intimately mixed with the encapsulation material to obtain a softer matrix, the softener may sometimes be regarded as part of the encapsulation material or matrix, which encapsulates the small particles of active ingredient.

When preparing the compressible chewing gum composition according to the invention, the particles of one or more encapsulation delivery systems are blended with the chewing gum granules and further particulated chewing gum ingredients. The obtained blend, that is the compressible chewing gum composition, is compressed in a tabletting press according to the methods known in the art. One of numerous methods is for example described in WO2004006686, hereby incorporated by reference.

According to the invention combinations of encapsulation delivery systems in the compressible chewing gum composition may be applied to obtain, not only a delayed or prolonged release, but a controlled release. A modified or controlled release may involve prolonged release of some ingredients, and relatively faster release of other ingredients, which may be obtained by application of different encapsulation delivery systems. Also, it may be desired in a controlled release to combine a quick release and a prolonged release of the same ingredient, by locating this same ingredient in different encapsulation delivery systems.

As natural resin has shown advantageous properties as encapsulation material in a compressible chewing gum composition according to the invention, new possibilities of modifying and controlling the release in compressed chewing gum are obtained.

As an example, two or more encapsulation delivery systems may be combined in a compressible chewing gum composition, a first encapsulation delivery system comprising an encapsulation material including at least one polyterpene resin and a second encapsulation delivery system comprising an encapsulation material including at least one polyvinyl acetate, one of the encapsulation delivery systems is a mixture of natural resin and polyvinyl acetate as encapsulation material.

Advantageous embodiments according to the invention may be obtained when the encapsulation delivery systems, if more than one, provide different release properties.

The encapsulation delivery systems may comprise at least one fast release encapsulation delivery system releasing 80-100% by weight of its active ingredient within the first 6 minutes of chewing the compressed chewing gum.

Alternatively, or as a supplement, the encapsulation delivery systems may also comprise at least one encapsulation delivery system releasing 60-80% by weight of its active ingredient within the first 6 minutes of chewing the compressed chewing gum.

Alternatively, or as a supplement, the encapsulation delivery systems may also comprise at least one encapsulation delivery system releasing 30-60% by weight of its active ingredient within the first 6 minutes of chewing the compressed chewing gum.

Natural resins comprised in the encapsulation delivery systems applied in the compressible chewing gum composition according to the invention may include, but are not limited to, natural rosin esters, often referred to as ester gums including for example glycerol esters of partially hydrogenated rosins, glycerol esters of polymerised rosins, glycerol esters of partially dimerised rosins, glycerol esters of tally oil rosins, pentaerythritol esters of partially hydrogenated rosins, methyl esters of rosins, partially hydrogenated methyl esters of rosins and pentaerythritol esters of rosins.

Natural resins such as hydrogenated resins or polymerised resins are well-known to the person skilled in the art and may e.g. be based on abietic acid.

In a preferred embodiment, the at least one natural resin comprised in the one or more encapsulation delivery systems may comprise at least one polyterpene resin. The polyterpene resins may be selected from polymerized monoterpenes, polymerized cyclic monoterpenes, polymerized delta-limonene, polymerized alpha-pinene, polymerized beta-pinene, and styrenated polyterpene. The polyterpene resins may typically have softening points in the range of 70°C to 150°C.

The at least one natural resin may moreover comprise at least one hydrogenated resin. Also, the at least one natural resin may comprise at least one polymerised resin. It is also envisioned that the natural resin may comprises mixtures of natural resins, such as at least one hydrogenated resin and at least one polyterpene resin; at least one polymerised resin and at least one polyterpene resin; at least one hydrogenated resin and at least one polymerised resin; or at least one hydrogenated resin, at least one polyterpene resin, and at least one polymerised resin.

The one or more encapsulation delivery systems may comprise a combination of two or more polyterpene resins. For example the encapsulation delivery system may comprise a combination of polymerised alpha-pinene and polymerised beta-pinene; a combination of polymerised alpha-pinene and polymerised limonene; a combination of polymerised alpha-pinene and styrenated polyterpene resin.

Some examples of suitable polyterpene resins include for example Piccolyte^{®} C 85, Delta - Limonene with softening point (Sp.) 82 - 88°C, Piccolyte^{®} C105, Delta - Limonene Sp. 97 - 103°C, Piccolyte^{®} C115, Delta - Limonene Sp. 112 - 118°C, Piccolyte^{®} C135, Delta - Limonene Sp. 130 - 136°C, Piccolyte^{®} A115, Alpha-pinen Sp. 112 - 118°C, Piccolyte^{®} HM115, Styrenated polyterpen resin Sp. 112 - 118, and Piccolyte^{®} S125, Beta - pinen Sp. 122 -128.

Examples of polymerised resins and hydrogenated resins include glycerol ester of polymerised gum rosin (softening point of 95°C - 105°C), and glycerol ester of partially hydrogenated gum rosin (softening point 77°C - 81 °C).

At least one of the one or more encapsulation delivery systems comprise polyvinyl acetate. In some embodiments, polyvinyl acetate (PVA) may e.g. have a GPC average molecular weight (Mw) in the range of 2,000 to 90,000 g/mol such as the range of 3,000 to 80,000 including the range of 30,000 to 50,000. Both low and high molecular weight polyvinyl acetate may be applicable, such as a low molecular weight PVA with Mw in the range of 10,000 - 15,000 or a high molecular weight PVA with Mw in the range of 50.000-70,000.

In some embodiments of the invention, the one or more encapsulation delivery systems further comprise one or more elastomers, which may e.g. be selected from the following: butyl rubber, polyisobutylen, isobutylene-isoprene copolymer, styrene-butadiene copolymer, styrene-isoprene-styrene copolymer, polyisoprene, polyethylene, vinyl acetate-vinyl laurate copolymer, and combinations thereof. If elastomers are used, the preferred elastomers may in some embodiments be butyl rubber and polyisobutylen, which e.g. may be used separately or in combination.

In preferred embodiments of the invention, however, elastomers are at least substantially avoided, as they provide elastomeric properties to the system, which are often not desired in the encapsulation delivery system according to the invention. The elastomers may influence the tensile strength undesirably.

When one or more softeners are applied in the encapsulation delivery system, they may typically be present in a total amount of 1-15% by weight of encapsulation delivery system.

The softening system comprised in the one or more encapsulation delivery systems may comprise a wax. The wax may be selected from the group consisting paraffin wax, bee*s wax, vegetable wax, candelilla wax, canauba wax, petroleum waxes, and the like, and mixtures thereof.

In a preferred embodiment of the invention, the wax has a high melting point e.g. a melting point in the range 70-100°C. Preferably, the wax is a microcrystalline wax.

The softening system comprised in the one or more encapsulation delivery systems may comprise a fat.

The fat is preferably a high melting fat, e.g. having a melting point in the range 30-100°C.

The fat may e.g. include partially or fully hydrogenated vegetable or animal fats, such as partially or fully hydrogenated coconut oil, partially or fully hydrogenated palm oil, partially or fully hydrogenated palm kernel oil, partially or fully hydrogenated rapeseed oil, partially or fully hydrogenated castor oil, partially or fully hydrogenated maize oil, partially or fully hydrogenated cottonseed oil, partially or fully hydrogenated olive oil, partially or fully hydrogenated sunflower oil, partially or fully hydrogenated safflower oil, partially or fully hydrogenated sesame oil, partially or fully hydrogenated soybean oil, beef tallow, partially or fully hydrogenated beef tallow, lard, and partially or fully hydrogenated lard, and any mixture thereof and any derivative thereof.

Further applicable softeners for an encapsulation delivery system may comprise emulsifiers. A number of different emulsifiers may be used, such as anionic, cationic, amphoteric or non-ionic emulsifiers. Further suitable emulsifiers may include polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), lecithins, sodium stearoyllatylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters. Some of the most suitable emulsifiers may be mono-diglyderides and triacetin.

The one or more encapsulation delivery systems may furthermore comprise a detackifier. The detackifier may e.g. be selected from the group consisting of talc powder, calcium carbonate, starches, such as corn starch; and mineral fillers, such as titanium dioxide.

The tensile strength of the encapsulation delivery system should preferably be in the range of 0.9 - 30 Pa, preferably in the range of 1.2 - 22 Pa.

As used herein, the term "tensile strength" includes the maximum stress a material subjected to a stretching load can withstand without tearing. A standard method for measuring tensile strength of a given substance is defined by the American Society of Testing Materials in method number ASTM-D638.

The active ingredients, which may be encapsulated in the encapsulation delivery system applied in the compressible chewing gum composition according to the invention, includes pharmaceutical active ingredients, nutraceutical active ingredients, high intensity sweeteners, food acids, flavours, cooling agents, warming agents, or any mixture thereof.

A range of different particle sizes of the one or more encapsulation delivery systems are envisioned. However, the average particle size of the one or more encapsulation delivery systems is typically in the range of 100-2000 µm, preferably in the range of 200-1000 µm, and even more preferred in the range of 500-1000 µm. The size of a particle is measured as the length of the longest dimension of the particle.

In a preferred embodiment of the invention, the average particle size of the at least one active ingredient in the encapsulation delivery system is in the range of 0.1-100 µm, preferably in the range of 1-50 µm.

The compressible chewing gum composition and compressed chewing gum according to the invention may have an increased stability relative to prior art compressed chewing gum. The increased stability is at least partly obtained as a result of the encapsulation delivery system. Without being bound by theory, it is believed that the increased stability is related to the presence of the natural resin, and in particular polyterpene resins, which have lower water permeability than prior art encapsulation materials.

Active ingredients may advantageously be applied in a chewing gum according to the invention. Active ingredients generally refer to those ingredients that are included in a delivery system and/or compressible chewing gum composition for the desired end benefit they provide to the user. In some embodiments, active ingredients can include medicaments, nutrients, nutraceuticals, herbals, nutritional supplements, pharmaceuticals, drugs, and the like and combinations thereof. Moreover, in the present context, active ingredients may refer to flavor components, high-intensity sweeteners or other taste establishing components.

Active ingredients may be classified according to The Anatomical Therapeutic Chemical (ATC) classification system, which is a system for classification of medicinal products according to their primary constituent and to the organ or system on which they act and their chemical, pharmacological and therapeutic properties.

The first level of the ATC is split into 14 main groups based on the anatomical group:
A: Alimentary tract and metabolism
B: Blood and blood forming organs
C: Cardiovascular system
D: Dermatologicals
G: Genito urinary system and sex hormones
H: Systemic hormonal preparations, excl. sex hormones and insulins
J: Antiinfectives for systemic use
L: Antineoplastic and immunomodulating agents
M: Musculo-skeletal system
N: Nervous system
P: Antiparasitic products, insecticides and repellents
R: Respiratory system
S: Sensory organs
V: Various

Further subdivision is done into a second, third, fourth and fifth sub-group, which is based on the therapeutic main group, the therapeutic/pharmacological subgroup, the chemical/therapeutic/pharmacological subgroup, and the chemical substance subgroup respectively. In this sense each active ingredient has been given a unique ATC identification code indicating where the active ingredient may be useful.

However, as some active ingredients are useful in more than one area, some of the active ingredients mentioned in this document belong to two or more of the mentioned groups, e.g. phenylephrine, which has an ATC identification code in both C, R, and S, i.e. both C01CA06, R01AA04, R01AB01, R01BA03, S01FB01, and S01GA05 are ATC identification codes identifying phenylephrine.

The following list discloses examples of active ingredients which can be classified according to the ATC classification mentioned above and which are active ingredients which may be used in a chewing gum granule or a compressed chewing gum according to the invention:
Ephedrine, Magaldrate, Pseudoephedrine, Sildenafil, Xylocaine, Benzalconium chloride, Caffeine, - Phenylephrine, Amfepramone, Orlistat, Sibutramine, Acetaminophen, Aspirin, Aluminum amino acetate, Aluminum amino acetate in combination with Magnesium oxide, Aluminum oxide hydrate in combination with Magnesiumoxide, Calcium carbonate in combination with Magnesium hydroxide, Calciumcarbonate, Dihydroxy Aluminum sodium carbonate, Magnesiumoxide, Glitazones, Metformin, Chlorpromazine, Dimenhydrinat, Domperidone, Meclozine, Metoclopramide, Odansetron, Prednisolone, Promethazine, Acrivastine, Cetirizine, Cinnarizine, Clemastine, Cyclizine, Desloratadine, Dexchlorpheniramine, Dimenhydrinate, Ebastine, Fexofenadine, Ibuprofen, Levolevoproricin, Loratadine, Meclozine, Mizolastine, Promethazine, Miconazole, Vitamin B12, Folic acid, Ferro compounds, vitamin C, Chlorhexidine diacetate, Fluoride, Decapeptide KSL, Aluminum fluoride, Aminochelated calcium, Ammonium fluoride, Ammonium fluorosilicate, Ammonium monofluorphosphate, Calcium fluoride, Calcium gluconate, Calcium glycerophosphate, Calcium lactate, Calcium monofluorphosphate, Calciumcarbonate, Carbamide, Cetyl pyridinium chloride, Chlorhexidine, Chlorhexidine digluconate, Chlorhexidine Chloride, Chlorhexidine diacetate, CPP Caseine Phospho Peptide; Hexetedine, Octadecentyl Ammonium fluoride, Potasium fluorosilicate, Potassium Chloride, Potassium monofluorphosphate, Sodium bicarbonate, Sodium carbonate, Sodium fluoride, Sodium fluorosilicate, Sodium monofluorphosphate, Sodium tri polyphosphate, Stannous fluoride, Stearyl Trihydroxyethyl Propylenediamine Dihydrofluoride, Strontium chloride, Tetra potassium pyrophosphate, Tetra sodium pyrophosphate, Tripotassium orthophosphate, Trisodium orthophosphate, Alginic acid, Aluminum hydroxide, Sodium bicarbonate, Sildenafil, Tadalafil, Vardenafil, Yohimbine, Cimetidine, Nizatidine, Ranitidine, Acetylsalicylic acid, Clopidogrel, Acetylcysteine, Bromhexine, Codeine, Dextromethorphan, Diphenhydramine, Noscapine, Phenylpropanolamine, vitamin D, Simvastatin, Bisacodyl, Lactitol, Lactulose, Magnesium oxide, Sodium picosulfate, Senna glycosides, Benzocaine, Lidocaine, Tetracaine, Almotriptan, Eletriptan, Naratriptan, Rizatriptan, Sumatriptan, Zolmitriptan, Calcium, Chromium, Copper, Iodine, Iron, Magnesium, Manganese, Molybdenium, Phosphor, Selenium, Zinc, Nicotine, Nicotine bitartrate, Nicotine pftalate, Nicotine polacrilex, Nicotine sulphate, Nicotine tartrate, Nicotine citrate, Nicotine lactate, Chloramine, Hydrogenperoxide, Metronidazole, Triamcinolonacetonide, Benzethonium Chl., Cetyl pyrid. Chl., Chlorhexidine, Fluoride, Lidocaine, Amphotericin, Miconazole, Nystatin, Fish oil, Ginkgo Biloba, Ginseng, Ginger, Purple coneflower, Saw Palmetto, Cetirizine, Levocetirizine, Loratadine, Diclofenac, Flurbiprofen, Acrivastine Pseudoephedrine, Loratadine Pseudoephedrine, Glucosamine, hyaluronic acid, Decapeptide KSL-W, Decapeptide KSL, Resveratrol, Misoprostol, Bupropion, Nicotine, Ondansetron HCl, Esomeprazole, Lansoprazole, Omeprazole, Pantoprazole, Rabeprazole, Bacteria and the like, Loperamide, Simethicone, Acetylsalicylic acid and others, Sucralfate, Vitamin A, Vitamin B1, Vitamin B12, Vitamin B2, Vitamin B6, Biotin, Vitamin C, Vitamin D, Vitamin E, Folinic acid, Vitamin K, Niacin, Q10, Clotrimazole, Fluconazole, Itraconazole, Ketoconazole, Terbinafine, Allopurinol, Probenecid, Atorvastatin, Fluvastatin, Lovastatin, Nicotinic acid, Pravastatin, Rosuvastatin, Simvastatin, Pilocarpine, Naproxen, Alendronate, Etidronate, Raloxifene, Risedronate, Benzodiazepines, Disulfiram, Naltrexone, Buprenorphine, Codeine, Dextropropoxyphene, Fentanyl, Hydromorphone, Ketobemidone, Ketoprofen, Methadone, Morphine, Naproxen, Nicomorphine, Oxycodone, Pethidine, Tramadol, Amoxicillin, Ampicillin, Azithromycin, Ciprofloxacin, Clarithromycin, Doxycyclin, Erythromycin, Fusidic acid, Lymecycline, Metronidazole, Moxifloxacin, Ofloxacin, Oxytetracycline, Phenoxymethylpenicillin, Rifamycins, Roxithromycin, Sulfamethizole, Tetracycline, Trimethoprim, Vancomycin, Acarbose, Glibenclamide, Gliclazide, Glimepiride, Glipizide, Insulin, Repaglinide, Tolbutamide, Oseltamivir, Aciclovir, Famciclovir, Penciclovir, Valganciclovir, Amlopidine, Diltiazem, Felodipine, Nifedipine, Verapamil, Finasteride, Minoxidil, Cocaine, Buphrenorphin, Clonidine, Methadone, Naltrexone, Calciumantagonists, Clonidine, Ergotamine, β-blockers, Aceclofenac, Celecoxib, Dexiprofen, Etodolac, Indometacin, Ketoprofen, Ketorolac, Lornoxicam, Meloxicam, Nabumetone, Oiroxicam, Parecoxib, Phenylbutazone, Piroxicam, Tiaprofenic acid, Tolfenamic acid, Aripiprazole, Chlorpromazine, Chlorprothixene, Clozapine, Flupentixol, Fluphenazine, Haloperidol, Lithium carbonate, Lithium citrate, Melperone, Penfluridol, Periciazine, Perphenazine, Pimozide, Pipamperone, Prochlorperazine, Risperidone, Thioridizin, Fluconazole, Itraconazole, Ketoconazole, Voriconazole, Opium, Benzodiazepines, Hydroxine, Meprobamate, Phenothiazine, Aluminiumaminoacetate, Esomeprazole, Famotidine, Magnesium oxide, Nizatide, Omeprazole, Pantoprazole, Fluconazole, Itraconazole, Ketoconazole, Metronidazole, Amphetamine, Atenolol, Bisoprolol fumarate, Metoprolol, Metropolol, Pindolol, Propranolol, Auranofin, and Bendazac.

Further examples of useful active ingredients include active ingredients selected from the therapeutical groups comprising: Analgesic, Anaestetic, Antipyretic, Anti allergic, Anti-arrytmic, Appetite suppressant, Antifungal, Anti-inflammatory, Broncho dilator, Cardiovascular drugs, Coronary dilator, Cerebral dilator, Peripheral vasodilator, Anti-infective, Psychotropic, Anti- manic, Stimulant, Antihistamine, Laxative, Decongestrant, Gastro-intestinal sedative, Sexual dysfunction agent, Desinfectants, Anti-diarrheal, Anti-anginal substance, Vasodilator, Anti-hypertensive agent, Vasoconstrictor, Migraine treating agent, Antibiotic, Tranquilizer, Ntipsychotic, Anti-tumor drug, Anticoagulant, Antithrombotic agent, Hypnotic, Sedative, Anti-emetic, Anti-, auseant, Anticonvulsant, Neuromuscular agent, Hyper and hypoglycaemic, Thyroid and antithyroid, Diuretic, Antispasmodic, Uterine relaxant, Anti-obesity agent, Anoretic, Spasnolytics, Anabolic agent, Erythropoietic agent, Anti-asthmatic, Expectorant, Cough suppressant, Mucolytic, Anti-uricemic agent, Dental vehicle, Breath freshener, Antacid, Anti-diuretc, Anti-flatulent, Betablokker, Teeth Whitener, Enzyme, Co-enzyme, Protein, Energy booster, Fiber, Probiotics, Prebiotics, Antimicrobial agent, NSAID, Anti-tussives, Decongestrants, Anti-histamines, Expectorants, Anti-diarrheals, Hydrogen antagonists, Proton pump inhibitors, General nonselective CNS depressants, General nonselective CNS stimulants, Selectively CNS function modyfying drugs, Antiparkinsonism, Narcotic-analgetics, Analgetic-antipyretics, Psychopharmacological drugs, and Sexual dysfunction agents.

Examples of useful active ingredients include: Casein glyco-macro-peptide (CGMP), Nicotine, Nicotine bitartrate, Nicotine sulphate, Nicotine tartrate, Nicotine pftalate, Nicotine lactate, Nicotinecitrate, Nicotine polacrilex, Triclosan, Cetyl pyridinium chloride, Domiphen bromide, Quaternary ammonium salts, Zinc components, Sanguinarine, Fluorides, Alexidine, Octonidine, EDTA, Aspirin, Acetaminophen, Ibuprofen, Ketoprofen, Diflunisal, Fenoprofen calcium, Naproxen, Tolmetin sodium, Indomethacin, Benzonatate, Caramiphen edisylate, Menthol, Dextromethorphan hydrobromide, Theobromine hydrochloride, Chlophendianol Hydrochloride, Pseudoephedrine Hydrochloride, Phenylephrine, Phenylpropanolamine, Pseudoephedrine sulphate, Brompheniramine maleate, Chlorpheniramine- maleate, Carbinoxamine maleate, Clemastine fumarate, Dexchlorpheniramine maleate, Dephenhydramine hydrochloride, Diphenpyralide hydrochloride, Azatadine maleate, Diphenhydramine citrate, Doxylamine succinate, Promethazine hydrochloride, Pyrilamine maleate, Tripellenamine citrate, Triprolidine hydrochloride, Acrivastine, Loratadine, Brompheniramine, Dexbrompheniamine, Guaifenesin, Ipecac, Potassium iodide, Terpin hydrate, Loperamide, Famotidine, Ranitidine, Omeprazole, Lansoprazole, Aliphatic alcohols, Barbiturates, Caffeine, Nicotine, Strychnine, Picrotoxin, Pentyenetetrazol, Phenyhydantoin, Phenobarbital, Primidone, Carbamazapine, Etoxsuximide, Methsuximide, Phensuximide, Trimethadione, Diazepam, Benzodiazepines, Phenacemide, Pheneturide, Acetazolamide, Sulthiame, Bromide, Levodopa, Amantadine, Morphine, Heroin, Hydromorphone, Metopon, Oxymorphone, Levophanol, Codeine, Hydrocodone, Xycodone, Nalorphine, Naloxone, Naltrexone, Salicylates, Phenylbutazone, Indomethacin, Phenacetin, Chlorpromazine, Methotrimeprazine, Haloperidol, Clozapine, Reserpine, Imipramine, Tranylcypromine, Phenelzine, Lithium, Sildenafil citrate, Tadalafil, and Vardenafil CL.

Examples of useful active ingredients include active ingredients selected from the groups of ace-inhibitors, antianginal drugs, anti- arrhythmias, anti-asthmatics, anti-cholesterolemics, analgesics, anesthetics, anticonvulsants, anti-depressants, antidiabetic agents, anti-diarrhea preparations, antidotes, anti-histamines, anti-hypertensive drugs, anti-inflammatory agents, anti-lipid agents, anti- manics, antinauseants, anti-stroke agents, anti-thyroid preparations, anti-tumor drugs, anti- viral agents, acne drugs, alkaloids, amino acid preparations, anti-tussives, anti- uricemic drugs, anti- viral drugs, anabolic preparations, systemic and non-systemic anti-infective agents, anti-neoplasties, antiparkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies such as sildenafil citrate, which is currently marketed as Viagra™, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocriptine or nicotine, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anti-coagulants, anti-thrombotic drugs, hypnotics, anti-emetics, antinauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Examples of active ingredients contemplated for use in the present invention can include antacids, H2-antagonists, and analgesics. For example, antacid dosages can be prepared using the ingredients calcium carbonate alone or in combination with magnesium hydroxide, and/or aluminum hydroxide. Moreover, antacids can be used in combination with H2-antagonists.

Analgesics include opiates and opiate derivatives, such as Oxycontin™, ibuprofen, aspirin, acetaminophen, and combinations thereof that may optionally include caffeine.

Other drug active ingredients for use in embodiments can include anti-diarrheals such as Immodium™ AD, anti-histamines, anti-tussives, decongestants, vitamins, and breath fresheners. Also contemplated for use herein are anxiolytics such as Xanax™; anti-psychotics such as Clozaril™ and Haldol™; non-steroidal antiinflammatories (NSAID's) such as ibuprofen, naproxen sodium, Voltaren™ and Lodine™, anti-histamines such as Claritin™, Hismanal™, Relafen™, and Tavist™; antiemetics such as Kytril™ and Cesamet™; bronchodilators such as Bentolin™, Proventil™; anti-depressants such as Prozac™, Zoloft™, and Paxil™; anti-migraines such as Imigra™, ACE-inhibitors such as Vasotec™, Capoten™ and Zestril™; anti-Alzheimer's agents, such as Nicergoline™; and CaH-antagonists such as Procardia™, Adalat™, and Calan™.

The popular H2-antagonists which are contemplated for use in the present invention include cimetidine, ranitidine hydrochloride, famotidine, nizatidien, ebrotidine, mifentidine, roxatidine, pisatidine and aceroxatidine.

Active antacid ingredients can include, but are not limited to, the following:
aluminum hydroxide, dihydroxyaluminum aminoacetate, aminoacetic acid, aluminum phosphate, dihydroxyaluminum sodium carbonate, bicarbonate, bismuth aluminate, bismuth carbonate, bismuth subcarbonate, bismuth subgallate, bismuth subnitrate, bismuth subsilysilate, calcium carbonate, calcium phosphate, citrate ion (acid or salt), amino acetic acid, hydrate magnesium aluminate sulfate, magaldrate, magnesium aluminosilicate, magnesium carbonate, magnesium glycinate, magnesium hydroxide, magnesium oxide, magnesium trisilicate, milk solids, aluminum mono-ordibasic calcium phosphate, tricalcium phosphate, potassium bicarbonate, sodium tartrate, sodium bicarbonate, magnesium aluminosilicates, tartaric acids and salts. A variety of nutritional supplements may also be used as active ingredients including virtually any vitamin or mineral. For example, vitamin A, vitamin C, vitamin D, vitamin E, vitamin K, vitamin B6, vitamin B12, thiamine, riboflavin, biotin, folic acid, niacin, pantothenic acid, sodium, potassium, calcium, magnesium, phosphorus, sulfur, chlorine, iron, copper, iodine, zinc, selenium, manganese, choline, chromium, molybdenum, fluorine, cobalt and combinations thereof, may be used. Examples of nutritional supplements that can be used as active ingredients are set forth in U.S. Patent Application Publication Nos. 2003/0157213 A1, 2003/0206993 and 2003/0099741 A1 which are incorporated in their entirety herein by reference for all purposes. Various herbals may also be used as active ingredients such as those with various medicinal or dietary supplement properties. Herbals are generally aromatic plants or plant parts and or extracts thereof that can be used medicinally or for flavoring. Suitable herbals can be used singly or in various mixtures. Commonly used herbs include Echinacea, Goldenseal, Calendula, Rosemary, Thyme, Kava Kava, Aloe, Blood Root, Grapefruit Seed Extract, Black Cohosh, Ginseng, Guarana, Cranberry, Ginko Biloba, St. John's Wort, Evening Primrose Oil, Yohimbe Bark, Green Tea, Ma Huang, Maca, Bilberry, Lutein, and combinations thereof.

Especially when hydrophilic, encapsulation of the active will result in a delay in the release of the predominant amount of the active during consumption of a compressible chewing gum that includes the encapsulated active (e.g., as part of a delivery system added as an ingredient to the compressible chewing gum), in some embodiments, the release profile of the ingredient (e.g., the active) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more components of an effervescing system are managed for a compressible gum. The effervescent system may include one or more edible acids and one or more edible alkaline materials. The edible acid(s) and the edible alkaline material(s) may react together to generate effervescence. In some embodiments, the alkaline material(s) may be selected from, but is not limited to, alkali metal carbonates, alkali metal bicarbonates, alkaline earth metal carbonates, alkaline earth metal bicarbonates, and combinations thereof. The edible acid(s) may be selected from, but is not limited to, citric acid, phosphoric acid, tartaric acid, malic acid, ascorbic acid, and combinations thereof. In some embodiments, an effervescing system may include one or more other ingredients such as, for example, carbon dioxide, oral care ingredients, flavorants, etc.

For examples of use of an effervescing system in a chewing gum, refer to U.S. Provisional Patent No. 60/618,222 filed October 13, 2004, and entitled "Effervescent Pressed Gum Tablet Compositions," the contents of which are incorporated herein by reference for all purposes. Other examples can be found in U.S. Patent No. 6,235,318, the contents of which are incorporated herein by reference for all purposes. Typically, encapsulation of the one or more ingredients in an effervescing system will result in a delay in the release of the predominant amount of the one or more ingredients during consumption of a compressible chewing gum that includes the encapsulated one or more ingredients (e.g., as part of a delivery system added as an ingredient to the compressible chewing gum composition). The release profile of the one or more ingredients can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more appetite suppressors are managed for a compressible gum. Appetite suppressors can be ingredients such as fiber and protein that function to depress the desire to consume food. Appetite suppressors can also include benzphetamine, diethylpropion, mazindol, phendimetrazine, phentermine, hoodia (P57), Olibra™, ephedra, caffeine and combinations thereof. Appetite suppressors are also known by the following trade, names: Adipex™, Adipost™, Bontril™ PDM, Bontril™ Slow Release, Didrex™, Fastin™, Ionamin™, Mazanor™, Melfiat™, Obenix™, Phendiet™, Phendiet-105™, Phentercot™, Phentride™, Plegine™, Prelu-2™, Pro-Fast™, PT 105™, Sanorex™, Tenuate™, Sanorex™, Tenuate™, Tenuate Dospan™, Tepanil Ten-Tab™, Teramine™, and Zantryl™. These and other suitable appetite suppressors are further described in the following U.S. patents, all of which are incorporated in their entirety by reference hereto: U.S. 6,838,431 to Portman, U.S. 6,716,815 to Portman, U.S. 6,558,690 to Portman, U.S. 6,468,962 to Portman, U.S. 6,436,899 to Portman.

Typically, encapsulation of the appetite suppressor will result in a delay in the release of the predominant amount of the appetite suppressor during consumption of a compressible chewing gum that includes the encapsulated appetite suppressor (e.g., as part of a delivery system added as an ingredient to the compressible chewing gum). In some embodiments, the release profile of the ingredient (e.g., the appetite suppressor) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more breath fresheners are managed for a compressible gum. Breath fresheners can include essential oils as well as various aldehydes, alcohols, and similar materials. In some embodiments, essential oils can include oils of spearmint, peppermint, wintergreen, sassafras, chlorophyll, citral, geraniol, cardamom, clove, sage, carvacrol, eucalyptus, cardamom, magnolia bark extract, marjoram, cinnamon, lemon, lime; grapefruit, and orange. In some embodiments, aldehydes such as cinnamic aldehyde and salicylaldehyde can be used. Additionally, chemicals such as menthol, carvone, iso-garrigol, and anethole can function as breath fresheners. Of these, the most commonly employed are oils of peppermint, spearmint and chlorophyll.

In addition to essential oils and chemicals derived from them, in some embodiments, breath fresheners can include but are not limited to zinc citrate, zinc acetate, zinc fluoride, zinc ammonium sulfate, zinc bromide, zinc iodide, zinc chloride, zinc nitrate, zinc flurosilicate, zinc gluconate, zinc tartarate, zinc succinate, zinc formate, zinc chromate, zinc phenol sulfonate, zinc dithionate, zinc sulfate, siliver nitrate, zinc salicylate, zinc glycerophosphate, copper nitrate, chlorophyll, copper chlorophyll, chlorophyllin, hydrogenated cottonseed oil, chlorine dioxide, beta cyclodextrin, zeolite, silica-based materials, carbon-based materials, enzymes such as laccase, and combinations thereof. In some embodiments, the release profiles of probiotics can be managed for a compressible gum including, but not limited to lactic acid producing microorganisms such as Bacillus coagulans, Bacillus subtilis, Bacillus laterosporus, Bacillus laevolacticus, Sporolactobacillus inulinus, Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae, Leuconostoc mesenteroides, Bacillus coagulans, Bacillus subtilis, Bacillus laterosporus, Bacillus laevolacticus, Sporolactobacillus inulinus and mixtures thereof. Breath fresheners are also known by the following trade names: Retsyn™, Actizol™, and Nutrazin™. Examples of malodor-controlling compositions are also included in U.S. Patent No. 5,300,305 to Stapler et al. and in U.S. Patent Application Publication Nos. 2003/0215417 and 2004/0081713 which are incorporated in their entirety herein by reference for all purposes.

Typically, encapsulation of the breath-freshening ingredient will result in a delay in the release of the predominant amount of the active during consumption of a compressible chewing gum that includes the encapsulated breath-freshening ingredient (e.g., as part of a delivery system added as an ingredient to the compressible chewing gum composition). In some embodiments, the release profile of the ingredient (e.g., the breath-freshening ingredient) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more dental care ingredients may be managed for a compressible gum. Such dental care ingredients (also known as oral care ingredients) may include but are not limited to tooth whiteners, stain removers, oral cleaning, bleaching agents, desensitizing agents, dental remineralization agents, antibacterial agents, anticaries agents, plaque acid buffering agents, surfactants and anticalculus agents. Non-limiting examples of such ingredients can include, hydrolytic agents including proteolytic enzymes, abrasives such as hydrated silica, calcium carbonate, sodium bicarbonate and alumina, other active stain-removing components such as surface-active agents, including, but not limited to anionic surfactants such as sodium stearate, sodium palminate, sulfated butyl oleate, sodium oleate, salts of fumaric acid, glycerol, hydroxylated lecithin, sodium lauryl sulfate and chelators such as polyphosphates, which are typically employed as tartar control ingredients. In some embodiments, dental care ingredients can also include tetrasodium pyrophosphate and sodium tri-polyphosphate, sodium bicarbonate, sodium acid pyrophosphate, sodium tripolyphosphate, xylitol, sodium hexametaphosphate. In some embodiments, peroxides such as carbamide peroxide, calcium peroxide, magnesium peroxide, sodium peroxide, hydrogen peroxide, and peroxydiphospate are included. In some embodiments, potassium nitrate and potassium citrate are included. Other examples can include casein glycomacropeptide, calcium casein peptone-calcium phosphate, casein phosphopeptides, casein phosphopeptide- amorphous calcium phosphate (CPP-ACP), and amorphous calcium phosphate. Still other examples can include papaine, krillase, pepsin, trypsin, lysozyme, dextranase, mutanase, glycoamylase, amylase, glucose oxidase, and combinations thereof. Further examples can include surfactants such as sodium stearate, sodium ricinoleate, and sodium lauryl sulfate surfactants for use in some embodiments to achieve increased prophylactic action and to render the dental care ingredients more cosmetically acceptable. Surfactants can preferably be detersive materials which impart to the composition detersive and foaming properties. Suitable examples of surfactants are water-soluble salts of higher fatty acid monoglyceride monosulfates, such as the sodium salt of the monosulfated monoglyceride of hydgrogenated coconut oil fatty acids, higher alkyl sulfates such as sodium lauryl sulfate, alkyl aryl sulfonates such as sodium dodecyl benzene sulfonate, higher alkyl sulfoacetates, sodium lauryl sulfoacetate, higher fatty acid esters of 1,2-dihydroxy propane sulfonate, and the substantially saturated higher aliphatic acyl amides of lower aliphatic amino carboxylic acid compounds, such as those having 12 to 16 carbons in the fatty acid, alkyl or acyl radicals, and the like. Examples of the last mentioned amides are N-lauroyl sarcosine, and the sodium, potassium, and ethanolamine salts of N-lauroyl, N-myristoyl, or N-palmitoyl sarcosine. In addition to surfactants, dental care ingredients can include antibacterial agents such as, but not limited.to, triclosan, chlorhexidine, zinc citrate, silver nitrate, copper, limonene, and cetyl pyridinium chloride. In some embodiments, additional anticaries agents can include fluoride ions or fluorine-providing components such as inorganic fluoride salts. In some embodiments, soluble alkali metal salts, for example, sodium fluoride, potassium fluoride, sodium fluorosilicate, ammonium fluorosilicate, sodium monofluorophosphate, as well as tin fluorides, such as stannous fluoride and stannous chloride can be included. In some embodiments, a fluorine-containing compound having a beneficial effect on the care and hygiene of the oral cavity, e.g., diminution of enamel solubility in acid and protection of the teeth against decay may also be included as an ingredient. Examples thereof include sodium fluoride, stannous fluoride, potassium fluoride, potassium stannous fluoride (SnF.sub.2 -KF), sodium hexafluorostannate, stannous chlorofluoride, sodium fluorozirconate, and sodium monofluorophosphate. In some embodiments, urea is included. Further examples are included in the following U.S. patents and U.S. published patent applications, the contents of all of which are incorporated in their entirety herein by reference for all purposes: U.S. Patent Nos. 5,227,154 to Reynolds, 5,378,131 to Greenberg, 6,846,500 to Luo et al. 6,733,818 to Luo et al., 6,696,044 to Luo et al., 6,685,916 to Holme et al., 6,485,739 to Luo et al., 6,479,071 to Holme et al., 6,471,945 to Luo et al., U.S. Patent Publication Nos. 20050025721. to Holme et al., 2005008732 to Gebreselassie et al., and 20040136928 to Holme et al.

Typically, encapsulation of the active will result in a delay in the release of the predominant amount of the active during consumption of a compressible chewing gum that includes the encapsulated active (e.g., as part of a delivery system added as an ingredient to the compressible chewing gum composition). In some embodiments, the release profile of the ingredient (e.g., the dental care active) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more flavor potentiators can be managed for a compressible gum. Flavor potentiators can consist of materials that may intensify, supplement, modify or enhance the taste and/or aroma perception of an original material without introducing a characteristic taste and/or aroma perception of their own. In some embodiments, potentiators designed to intensify, supplement, modify, or enhance the perception of flavor, sweetness, tartness, umami, kokumi, saltiness and combinations thereof can be included. In some embodiments, sweetness may be potentiated by the inclusion of monoammonium glycyrrhizinate, licorice glycyrrhizinates, citrus aurantium, maltol, ethyl maltol, vanilla, vanillin, and combinations thereof. In some embodiments, sugar acids, sodium chloride, potassium chloride, sodium acid sulfate, and combinations thereof may be included for flavor potentiation. In other examples, glutamates such as monosodium glutamate (MSG), monopotassium glutamate, hydrolyzed vegetable protein, hydrolyzed animal protein, yeast extract, and combinations thereof are included. Further examples can include glutathione, and nucleotides such as inosine monophosphate (IMP), disodium inosinate, xanthosine monophosphate, guanylate monophosphate (GMP), and combinations thereof. For bitterness blocking or taste masking, ingredients that interact with bitterness receptors to suppress bitterness or off tastes may be included. In some embodiments, adenosine monophosphate (AMP) can be included for bitterness suppression. Bitterness modification can also be accomplished by using sweetness or flavors with complementary bitter notes such as chocolate. Further examples of flavor potentiator compositions that impart kokumi are also included in U.S. Patent No. 5,679,397 to Kuroda et al, the entire contents of which are incorporated in its entirety herein by reference.

Typically, encapsulation of a flavor potentiator will result in a delay in the release of the predominant amount of the flavor potentiator during consumption of a compressible chewing gum that includes the encapsulated flavor potentiator (e.g., as part of a delivery system added as an ingredient to the compressible chewing gum composition). In some embodiments, the release profile of the ingredient (e.g., the flavor potentiator) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more acids may be managed for a compressible gum. Acids can include, but are not limited to acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof.

Typically, encapsulation of a food acid will result in a delay in the release of the predominant amount of the active during consumption of a compressible chewing gum that includes the encapsulated food acid (e.g., as part of a delivery system added as an ingredient to the compressible chewing gum). In some embodiments, the release profile of the ingredient (e.g., the food acid) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more micronutrients can be managed for a compressible gum. Micronutrients can include materials that have an impact on the nutritional wellbeing of an organism even though the quantity required by the organism to have the desired effect is small relative to macronutrients such as protein, carbohydrate, and fat. Micronutrients can include, but are not limited to vitamins, minerals, enzymes, phytochemicals, antioxidants, and combinations thereof. In some embodiments, vitamins can include fat soluble vitamins such as vitamin A, vitamin D, vitamin E, and vitamin K and combinations thereof, in some embodiments, vitamins can include water soluble vitamins such as vitamin C (ascorbic acid), the B vitamins (thiamine or B1, riboflavoin or B2, niacin or B3, pyridoxine or B6, folic acid or B9, cyanocobalimin or B12, pantothenic acid, biotin), and combinations thereof.

In some embodiments, minerals can include but are not limited to sodium, magnesium, chromium, iodine, iron, manganese, calcium, copper, fluoride, potassium, phosphorous, molybdenum, selenium, zinc, and combinations thereof.

In some embodiments micronutrients can include but are not limited to L- carnitine, choline, coenzyme Q10, alpha-lipoic acid, omega-3 -fatty acids, pepsin, phytase, trypsin, lipases, proteases, cellulases, and combinations thereof.

Antioxidants can include materials that scavenge free radicals. In some embodiments, antioxidants can include but are not limited to ascorbic acid, citric acid, rosemary oil, vitamin A, vitamin E, vitamin E phosphate, tocopherols, di-alpha-tocopheryl phosphate, tocotrienols, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, flavonoids, and combinations thereof.

In some embodiments, phytochemicals can include but are not limited to cartotenoids, chlorophyll, chlorophyllin, fiber, flavanoids, anthocyanins, cyaniding, delphinidin, malvidin, pelargonidin, peonidin, petunidin, flavanols, catechin, epicatechin, epigallocatechin, epigallocatechingallate, theaflavins, thearubigins, proanthocyanins, flavonols, quercetin, kaempferol, myricetin, isorhamnetin, flavononeshesperetin, naringenin, eriodictyol, tangeretin, flavones, apigenin, luteolin, lignans, phytoestrogens, resveratrol, isoflavones, daidzein, genistein, glycitein, soy isoflavones, and combinations thereof.
Typically, encapsulation of the micronutrient will result in a delay in the release of the predominant amount of the active during consumption of a compressible chewing gum that includes the encapsulated micronutrient (e.g., as part of a delivery system added as an ingredient to the compressible chewing gum). In some embodiments, the release profile of the ingredient (e.g., the micronutrient) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more mouth moisteners can be managed for a compressible gum. Mouth moisteners can include, but are not limited to, saliva stimulators such as acids and salts and combinations thereof. In some embodiments, acids can include acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof. Mouth moisteners can also include hydrocolloid materials that hydrate and may adhere to oral surface to provide a sensation of mouth moistening. Hydrocolloid materials can include naturally occurring materials such as plant exudates, seed gums, and seaweed extracts or they can be chemically modified materials such as cellulose, starch, or natural gum derivatives. In some embodiments, hydrocolloid materials can include pectin, gum arabic, acacia gum, alginates, agar, carageenans, guar gum, xanthan gum, locust bean gum, gelatin, gellan gum, galactomannans, tragacanth gum, karaya gum, curdlan, konjac, chitosan, xyloglucan, beta glucan, furcellaran, gum ghatti, tamarin, bacterial gums, and combinations thereof. Additionally, in some embodiments, modified natural gums such as propylene glycol alginate, carboxymethyl locust bean gum, low methoxyl pectin, and their combinations can be included. In some embodiments, modified celluloses can be included such as microcrystalline cellulose, carboxymethlcellulose (CMC), methylcellulose (MC), hydroxypropylniethylcellulose (HPCM), and hydroxypropylcellulose (MPC), and combinations thereof. Similarly, humectants which can provide a perception of mouth hydration can be included. Such humectants can include, but are not limited to glycerol, sorbitol, polyethylene glycol, erythritol, and xylitol. Additionally, in some embodiments, fats can provide a perception of mouth moistening. Such fats can include medium chain triglycerides, vegetable oils, fish oils, mineral oils, and combinations thereof. Typically, encapsulation of a mouth moistening agent will result in a delay in the release of the predominant amount of the active during consumption of a compressible chewing gum that includes the encapsulated mouth moistening agent (e.g., as part of a delivery system added as an ingredient to the compressible chewing gum). In some embodiments, the release profile of the ingredient (e.g., the mouth moistening agent) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more ingredients that soothe the throat can be managed for a compressible gum. Throat soothing ingredients can include analgesics, anesthetics, demulcents, antiseptic, and combinations thereof. In some embodiments, analgesics/anesthetics can include menthol, phenol, hexylresorcinol, benzocaine, dyclonine hydrochloride, benzyl alcohol, salicyl alcohol, and combinations thereof. In some embodiments, demulcents can include but are not limited to slippery elm bark, pectin, gelatin, and combinations thereof. In some embodiments, antiseptic ingredients can include cetylpyridinium chloride, domiphen bromide, dequalinium chloride, and combinations thereof.

In some embodiments, antitussive ingredients such as chlophedianol hydrochloride, codeine, codeine phosphate, codeine sulfate, dextromethorphan, dextromethorphan hydrobromide, diphenhydramine citrate, and diphenhydramine hydrochloride, and combinations thereof can be included.

In some embodiments, throat soothing agents such as honey, propolis, aloe vera, glycerine, menthol and combinations thereof can be included. In still other embodiments, cough suppressants can be included. Such cough suppressants can fall into two groups: those that alter the texture or production of phlegm such as mucolytics and expectorants; and those that suppress the coughing reflex such as codeine (narcotic cough suppressants), antihistamines, dextromethorphan and isoproterenol (non-narcotic cough suppressants). In some embodiments, ingredients from either or both groups can be included.

In still other embodiments, antitussives can include, but are not limited to, the group consisting of codeine, dextromethorphan, dextrorphan, diphenhydramine, hydrocodone, noscapine, oxycodone, pentoxyverine and combinations thereof. In some embodiments, antihistamines can include, but are not limited to, acrivastine, azatadine, brompheniramine, chlo[phi]heniramine, clemastine, cyproheptadine, dexbrompheniramine, dimenhydrinate, diphenhydramine, doxylamine, hydroxyzine, meclizine, phenindamine, phenyltoloxamine, promethazine, pyrilamine, tripelennamine, triprolidine and combinations thereof. In some embodiments, non-sedating antihistamines can include, but are not limited to, astemizole, cetirizine, ebastine, fexofenadine, loratidine, terfenadine, and combinations thereof.

In some embodiments, expectorants can include, but are not limited to, ammonium chloride, guaifenesin, ipecac fluid extract, potassium iodide and combinations thereof. In some embodiments, mucolytics can include, but are not limited to, acetylcycsteine, ambroxol, bromhexine and combinations thereof. In some embodiments, analgesic, antipyretic and anti-inflammatory agents can include, but are not limited to, acetaminophen, aspirin, diclofenac, diflunisal, etodolac, fenoprofen, flurbiprofen, ibuprofen, ketoprofen, ketorolac, nabumetone, naproxen, piroxicam, caffeine and mixtures thereof. In some embodiments, local anesthetics can include, but are not limited to, lidocaine, benzocaine, phenol, dyclonine, benzonotate and mixtures thereof. In some embodiments nasal decongestants and ingredients that provide the perception of nasal clearing can be included. In some embodiments, nasal decongestants can include but are not limited to phenylpropanolamine, pseudoephedrine, ephedrine, phenylephrine, oxymetazoline, and combinations thereof. In some embodiments ingredients that provide a perception of nasal clearing can include but are not limited to menthol, camphor, borneol, ephedrine, eucalyptus oil, peppermint oil, methyl salicylate, bornyl acetate, lavender oil, wasabi extracts, horseradish extracts, and combinations thereof. In some embodiments, a perception of nasal clearing can be provided by odoriferous essential oils, extracts from woods, gums, flowers and other botanicals, resins, animal secretions, and synthetic aromatic materials.

Typically, encapsulation of a throat care agent will result in a delay in the release of the predominant amount of the active during consumption of a compressible chewing gum that includes the encapsulated throat care agent (e.g. as part of a delivery system added as an ingredient to the compressible chewing gum). In some embodiments, the release profile of the ingredient (e.g. the dental care active) can be managed for a compressible gum by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, one or more colors can be included. As classified by the United States Food, Drug, and Cosmetic Act (21 C.F.R. 73), colors can include exempt from certification colors (sometimes referred to as natural even though they can be synthetically manufactured) and certified colors (sometimes referred to as artificial), or combinations thereof. In some embodiments, exempt from certification or natural colors can include, but are not limited to annatto extract, (E 160b), bixin, norbixin, astaxanthin, dehydrated beets (beet powder), beetroot red/betanin (E 162), ultramarine blue, canthaxanthin (E161g), cryptoxanthin (E161c), rubixanthin (E161d), violanxanthin (E161e), rhodoxanthin (E161f), caramel (E150(a-d)), β-apo-8'-carotenal (E160e), β-carotene (E160a), alpha carotene, gamma carotene, ethyl ester of beta-apo-8 carotenal (E160f), fiavoxanthin (E161a), lutein (E161b), cochineal extract (E120); carmine (E132), carmoisine/azorubirie (E122), sodium copper chlorophyllin (E141), chlorophyll (E140), toasted partially defatted cooked cottonseed flour, ferrous gluconate, ferrous lactate, grape color extract, grape skin extract (enocianina), anthocyanins (E163), haematococcus algae meal, synthetic iron oxide, iron oxides and hydroxides (E172), fruit juice, vegetable juice, dried algae meal, tagetes (Aztec marigold) meal and extract, carrot oil, corn endosperm oil, paprika, paprika oleoresin, phaffia yeast, riboflavin (E101), saffron, titanium dioxide, turmeric (E100), turmeric oleoresin, amaranth (E123), capsanthin/capsorbin (E160c), lycopene (E160d), and combinations thereof.

In some embodiments, certified colors can include, but are not limited to, FD&C blue #1, FD&C blue #2, FD&C green #3, FD&C red #3, FD&C red #40, FD&C yellow #5 and FD&C yellow #6, tartrazine (E102), quinoline yellow (E104), sunset yellow (E110), ponceau (E124), erythrosine (E127), patent blue V (E131), titanium dioxide (E171), aluminum (E173), silver (E174), gold (E175), pigment rubine/lithol rubine BK (E180), calcium carbonate (E170), carbon black (E153), black PN/brilliant black BN (E151), green S/acid brilliant green BS (E142), and combinations thereof. In some embodiments, certified colors can include FD&C aluminum lakes. These consist of the aluminum salts of FD&C dyes extended on an insoluble substrate of alumina hydrate. Additionally, in some embodiments, certified colors can be included as calcium salts. Typically, encapsulation of a color will result in a delay in the release of the predominant amount of the active during consumption of a compressible chewing gum that includes the encapsulated color (e.g., as part of a delivery system added as an ingredient to the compressible chewing gum). In some embodiments, the release profile of the ingredient (e.g., the color) can be managed by managing various characteristics of the ingredient, delivery system containing the ingredient, and/or the compressible chewing gum containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, a delivery system or compressible chewing gum may include two or more ingredients for which managed release from the compressible chewing gum during consumption of the compressible chewing gum is desired. In some embodiments, the ingredients may be encapsulated or otherwise included separately in different delivery systems. Alternatively, in some embodiments the ingredients may be encapsulated or otherwise included in the same delivery system. As another possibility, one or more of the ingredients may be free (e.g. unencapsulated) while one or more other ingredients may be encapsulated. A compressible chewing gum may include a group of ingredients for which managed release of the group during consumption of the compressible chewing gum is desired. Groups of two or more ingredients for which managed release from a compressible chewing gum during consumption of the compressible chewing gum may be desired include, but are not limited to: color and flavor, multiple flavors, multiple colors, cooling agent and flavor, warming agent and flavor, cooling agent and warming agent, cooling agent and high-intensity sweetener, warming agent and high-intensity sweetener, multiple cooling agents (e.g., WS-3 and WS-23, WS-3 and menthyl succinate), menthol and one or more cooling agents, menthol and one or more warming agents, multiple warming agents, high-intensity sweetener(s) and tooth whitening active(s), high-intensity sweetener(s) and breath-freshening active(s), an ingredient with some bitterness and a bitterness suppressor for the ingredient, multiple high-intensity sweeteners (e.g., ace-k and aspartame), multiple tooth whitening actives (e.g., an abrasive ingredient and an antimicrobial ingredient, a peroxide and a nitrate, a warming agent and a polyol, a cooling agent and a polyol, multiple polyols, a warming agent and micronutrient, a cooling agent and a micronutrient, a warming agent and a mouth moistening agent, a cooling agent and a mouth moistening agent, a warming agent and a throat care agent, a cooling agent and a throat care agent, a warming agent and a food acid, a cooling agent and food acid, a warming agent and an emulsifier/surfactant, a cooling agent and an emulsifier/surfactant, a warming agent and a color, a cooling agent and a color, a warming agent and a flavor potentiator, a cooling agent and a flavor potentiator, a warming agent with sweetness potentiator, a cooling agent with a sweetness potentiator, a warming agent and an appetite suppressant, a cooling agent and an appetite suppressant, a high-intensity sweetener and a flavor, a cooling agent and a teeth-whitening agent, a warming agent and a teeth-whitening agent, a warming agent and breath-freshening agent, a cooling agent and a breath-freshening agent, a cooling agent and an effervescing system, a warming agent and an effervescing system, a warming agent and an antimicrobial agent, a cooling agent and an antimicrobial agent, multiple anticalcums ingredients, multiple remineralization ingredients, multiple surfactants, remineralization ingredients with demineralization ingredients, acidic ingredients with acid buffering ingredients, anticalculus ingredients with antibacterial ingredients, remineralization ingredients with anticalculus ingredients, anticalculus ingredients with remineralization ingredients with antibacterial ingredients, surfactant ingredients with anticalculus ingredients, surfactant ingredients with antibacterial ingredients, surfactant ingredients with remineralization ingredients, surfactants with anticalculus ingredients with antibacterial ingredients, multiple types of vitamins or minerals, multiple micronutrients, multiple acids, multiple antimicrobial ingredients, multiple breath-freshening ingredients, breath-freshening ingredients and antimicrobial ingredients, multiple appetite suppressors, acids and bases that react to effervesce, a bitter compound with a high-intensity sweetener, a cooling agent and an appetite suppressant, a warming agent and an appetite suppressant, a high-intensity sweetener and an appetite suppressant, a high-intensity sweetener with an acid, a probiotic ingredient and a prebiotic ingredient, a vitamin and a mineral, a metabolic enhancement ingredient with a macronutrient, a metabolic enhancement ingredient with a micronutrient, an enzyme with a substrate, a high-intensity sweetener with a sweetness potentiator, a cooling compound with a cooling potentiator, a flavor with a flavor potentiator, a warming compound with a warming potentiator, a flavor with salt, a high-intensity sweetener with salt, an acid with salt, a cooling compound with salt, a warming compound with salt, a flavor with a surfactant, an astringent compound with an ingredient to provide a sensation of hydration, etc. In some embodiments, the multiple ingredients may be part of the same delivery system or may be part of different delivery systems. Different delivery systems may use the same or different encapsulating materials.

Typically, encapsulation of the multiple ingredients will result in a delay in the release of the predominant amount of the multiple ingredients during consumption of a compressible chewing gum that includes the encapsulated multiple ingredients (e.g. as part of a delivery system added as an ingredient to the compressible chewing gum). This may be particularly helpful in situations wherein separate encapsulation of the ingredients may cause them to release with different release profiles. For example, different high-intensity sweeteners may have different release profiles because they have different water solubilities or differences in other characteristics. Encapsulating them together may cause them to release more simultaneously.

In some embodiments, the release profile of the multiple ingredients can be managed for a compressible gum by managing various characteristics of the multiple ingredients, the delivery system containing the multiple ingredients, and/or the compressible chewing gum containing the delivery system and/or how the delivery system is made in a manner as previously discussed above.

The active ingredients mentioned above are meant as examples of active ingredients which could be applicable in a chewing gum granule or compressed chewing gum, however, this list should not be considered as exhaustive.

Active ingredients to be applied in tablets according to embodiments of the invention may be applied as such or be included or bonded in different ways, such as being part of an inclusion complex e.g. as described in US 5,866,179, which is hereby incorporated by reference. A resin-bonding of nicotine is described in e.g. WO 2006/000232 which is also incorporated herein by reference. Further conventional methods of applying active ingredients may obviously be applied within the scope of the invention.

Agents for controlling the pH in the mouth during chewing of the tablet may preferably be included in order to facilitate the release of certain active ingredients. For example, sodium carbonate may be included in the chewing gum tablet according to the invention in order to control the release of nicotine. Nicotine and sodium carbonate may preferably be located in different encapsulation delivery systems. Also, it may be preferred to locate at least a part of the nicotine external to the encapsulation delivery systems in order to facilitate a fast release of nicotine from the compressed chewing gum tablet during chewing.

The active ingredients may advantageously be applied in a gum base-containing module or a tablet-module substantially free of gum base depending on the applied type of active ingredient. If the active ingredient is of the pharmaceutical type, such ingredient may very often advantageously be comprised in a tablet module substantially free of gum base whereas taste relevant active ingredients advantageously may be added to the gum base-containing module and very often to both types of modules. The taste relevant active ingredient may both be added as separate particles which are mixed and compressed with gum base-containing particles in one module and it may be incorporated into gum base-containing granules.

In the manufacturing of the compressible chewing gum composition and the compressed chewing gum according to the invention, mixing of gum base and chewing gum mass and forming of chewing gum granules may be performed by any process known within the chewing gum art.

Here below, conditions and procedures for manufacturing of gum base, chewing gum, and chewing gum granules are described only by way of non-limiting examples.

One of various suitable processes for preparation of gum bases applicable in the compressed chewing gum according to the invention may involve adding elastomers, elastomer plasticizers, fillers, and on occasion vinyl polymers, to a heated (10°C - 120°C) sigma blade mixer, or any suitable mixer known within the chewing gum art. The initial amounts of ingredients comprising the initial mass may be determined by the working capacity of the mixing kettle in order to attain a proper consistency and by the degree of compounding desired to break down the elastomer and increase chain branching. The higher the level of filler at the start or selection of a filler having a certain particle size distribution, the higher the degree of compounding and thus more of the elastomeric chain crosslinking is broken, causing more branching of the elastomer and thus lower viscosity gum bases and thus softer final gum base and gum made from such a gum base. The longer the time of compounding, and the lower the molecular weight or softening point gum base ingredients, the lower the viscosity and firmness of the final gum base. Compounding typically begins to be effective once the ingredients have massed together. Anywhere from 15 minutes to 90 minutes may typically be the length of compounding time. Preferably, the time of compounding may be from 20 minutes to about 60 minutes. The amount of added elastomer plasticizer depends on the level of elastomer and filler present. If too much elastomer plasticizer is added, the initial mass becomes over-plasticized and not homogeneous. After the initial ingredients have massed homogeneously and compounded for the time desired, the balance of the gum base ingredients are added in a sequential manner until a completely homogeneous molten mass is attained. Typically, any remainder of elastomer, elastomer plasticizer, vinyl polymer and filler, may be added within 60 minutes after the initial compounding time. The filler and the elastomer plasticizer would typically be individually weighed and added in portions during this time. The optional waxes and the softeners are typically added after the elastomer and elastomer plasticizers and during the next 60 minutes. Then the mass is allowed to become homogeneous before discharging. Typical gum base processing times may vary from about one to about three hours, preferably from about 1 1/2 to 2 1/2 hours, depending on the formulation. The final mass temperature when discharged may be between 70°C and 130°C and preferably between 100°C and 120°C. The completed molten mass of gum base is emptied from the mixing kettle into coated or lined pans, extruded or cast into any desirable shape and allowed to cool and solidify.

Other suitable gum base mixing procedures may involve any conventional continuous or batch-wise procedures using any mixer known within the art of chewing gum production to be suitable for gum base mixing. Examples of suitable manufacturing processes for gum base mixing are described in WO2006/058536, WO02071860, US4,968,511, US 5,135,760, 4,187,320, US2004142066, US5571543 US2006188605, US2004131725, US2001007686, US5773053, WO9608157, GB2049705, US4187320, US2001050879, and WO 2005/063038.

The gum base may be stored and transported, if convenient or necessary. The gum base may be subjected to granulation and formed into granules, which are suitable as chewing gum granules in the compressible chewing gum composition according to the invention. Alternatively, before granulation, the gum base may enter into a chewing gum mixing procedure in which further chewing gum ingredients, mainly water soluble ingredients, are added. The resulting chewing gum is granulated to be applicable in the compressible chewing gum composition according to the invention.

Those skilled in the art will recognize that many variations of the above-described procedure may be followed, including a one-step process as disclosed in US 2004/0115305, wherein the molten gum base mass may be used directly in mixing of chewing gum.

After completion of the mixing procedure, the gum base, optionally including at least some chewing gum ingredients, is granulated to form the chewing gum granules suitable in the compressible chewing gum composition according to the invention.

A suitable continuous manufacturing process is disclosed in WO 2004/098305 hereby incorporated by reference, where gum base or chewing gum granules are provided by means of an extrusion process.

The chewing gum according to the present invention may also be provided with an outer coating, which may be a hard coating, a soft coating, a film coating, or a coating of any type that is known in the art, or a combination of such coatings. The coating may typically constitute 0.1 to 75% by weight of a coated chewing gum piece.

One preferred outer coating type is a hard coating, which term is including sugar coatings and sugar-free (or sugarless) coatings and combinations thereof. The object of hard coating is to obtain a sweet, crunchy layer, which is appreciated by the consumer and to protect the gum centers. In a typical process of providing the chewing gum centers with a protective sugar coating the gum centers are successively treated in suitable coating equipment with aqueous solutions of crystallizable sugar such as sucrose or dextrose, which, depending on the stage of coating reached, may contain other functional ingredients, e.g. fillers, colors, etc.

In one presently preferred embodiment, the coating agent applied in a hard coating process is a sugarless coating agent, e.g. a polyol including as examples sorbitol, maltitol, mannitol, xylitol, erythritol, lactitol and isomalt or e.g. a mono- di-saccharide including as example trehalose.

The applicable hard coating may be selected from the group comprising of sugar coating and a sugarless coating and a combination thereof. The hard coating may e.g. comprise 50 to 100% by weight of a polyol selected from the group consisting of sorbitol, maltitol, mannitol, xylitol, erythritol, lactitol and Isomalt and variations thereof. In an embodiment of the invention, the outer coating is an edible film comprising at least one component selected from the group consisting of an edible film-forming agent and a wax. The film-forming agent may e.g. be selected from the group comprising cellulose derivative, a modified starch, a dextrin, gelatine, shellac, gum arabic, zein, a vegetable gum, a synthetic polymer and any combination thereof. In an embodiment of the invention, the outer coating comprises at least one additive component selected from the group comprising of a binding agent, a moisture-absorbing component, a film-forming agent, a dispersing agent, an antisticking component, a bulking agent, a flavoring agent, a coloring agent, a pharmaceutically or cosmetically active component, a lipid component, a wax component, a sugar, an acid and an agent capable of accelerating the after-chewing degradation of the degradable polymer.

Or alternatively a sugar-free soft coating e.g. comprising alternately applying to the centers a syrup of a polyol or a mono- di-saccharide, including as examples sorbitol, maltitol, mannitol, xylitol, erythritol, lactitol, isomalt and trehalose.

In further useful embodiments, a film coating is provided by film-forming agents such as a cellulose derivative, a modified starch, a dextrin, gelatine, zein, shellec, gum arabic, a vegetable gum, a synthetic polymer, etc. or a combination thereof.

In an embodiment of the invention, the outer coating comprises at least one additive component selected from the group comprising a binding agent, a moisture-absorbing component, a film-forming agent, a dispersing agent, an antisticking component, a bulking agent, a flavoring agent, a coloring agent, a pharmaceutically or cosmetically active component, a lipid component, a wax component, a sugar, and an acid.

A coated chewing gum center according to the invention may have any form, shape or dimension that permits the chewing gum center to be coated using any conventional coating process.

It should however be noted that application of different coating should be done with care as compressed chewing gum tablets may be very affected by direct contact with moisture or water.

Different aspect of process parameters, compression materials and release properties are described in the co-pending applications "Multi-modular chewing gum tablet", "High volume compressed chewing gum tablet", "Compressed tablet comprising polyol" and "Chewing gum granules for compressed chewing gum" filed at the same date and hereby incorporated by reference.

The following examples are provided for illustration but not limitation of the invention.

### EXAMPLE 1

### Chewing gum granules

An extruder (Leistritz ZSE/BL 360 kw 104, available from Leistritz GmbH, Germany) extruded the composition through the die plate into the liquid-filled chamber (granulator A5 PAC 6, available from GALA GmbH, Germany). Descriptions of the extruder and the granulator may be found in e.g. WO 2004/098305, incorporated herein by reference.
Chewing gum granules according to the below formulations of Tables 1 and 2 were made by the above extruder by appropriate feeding to the relevant inlets.

**Table 1 - Composition of chewing gum granules**

| **Components** | **Percentage** |
|---|---|
| Polyvinyl acetate (low Mw) | 28 |
| Natural resin | 5 |
| Elastomer. | 21 |
| Filler | 17 |
| Colour | 1 |
| Softener | 25 |
| Menthol flavor | 3 |

**Table 2 - Composition of chewing gum granules containing high intensity sweetener.**

| **Components** | **Percentage** |
|---|---|
| Polyvinyl acetate (low Mw) | 28 |
| Natural resin | 5 |
| Elastomer | 20 |
| Filler | 17 |
| Colour | 1 |
| Softener | 25 |
| Menthol flavour | 3 |
| Aspartame | 1 |

It should be emphasized that the above applied manufacturing methods of chewing gum granules are only exemplary and although advantageous not mandatory. Thus, an alternative method for the manufacturing of gum base-containing granules may be a conventional process involving that an initial gum base-containing mass is cooled to a low temperature, preferably to a temperature below 0°C, and then mechanically particulated into small, relatively irregular gum base-containing granules. If desired, these particles may be sieved or further processed to obtain a homogeneous granule blend. An example of such alternative process is disclosed in WO 2004/073691 as applied in a multi-layer tablet, hereby incorporated by reference.

### EXAMPLE 2

### Encapsulation delivery systems

The preparation of encapsulation delivery systems may be performed by a batch process in a mixer or a continuous process in an extruder. In an extruder process, manufacturing time is typically reduced as compared to the batch-wise mixing process.

The following encapsulation delivery systems were prepared in a batch process using a z-blade mixer. In some of the formulations, elastomers and/or detackifiers (anti-caking or anti-agglomeration agent) are included. In preparation of these formulations, these components are added first to the mixer, and mixing is performed at about 120°C. Then subsequently natural resin, polyvinyl acetate, softeners and active ingredients according to the procedure described here below.

When elastomers are not present in the formulation, preparation of the encapsulation delivery systems is commenced by adding polyterpene resin (Piccolyte C85) and/or polyvinyl acetate (Mw 50,000-70,000) to the z-blade mixer, and mixing is performed at about 90-100°C dependent on the softening point of the applied natural resin. Mixing is continued for about half an hour before softeners are added and the mixing is continued for about 5-10 minutes. As the last component, the active ingredient is added, and mixing is continued typically for about 5-10 minutes until the active ingredient is distributed sufficiently in the matrix, i.e. in the encapsulation delivery system. The temperature is lowered to about 80°C and the encapsulation delivery system mixture is emptied from the mixing kettle and allowed to cool before it is ground to particles.

Each of the encapsulation delivery systems no. 10-39 were particulated in two portions, into particle sizes of 106-500 µm and 500-1000 µm, respectively.

Examples of compositions of encapsulation delivery systems are given in table 3, table 4, and table 5. For reasons of comparison, the load (i.e. the percentage) of active ingredient is the same in each of the provided examples:

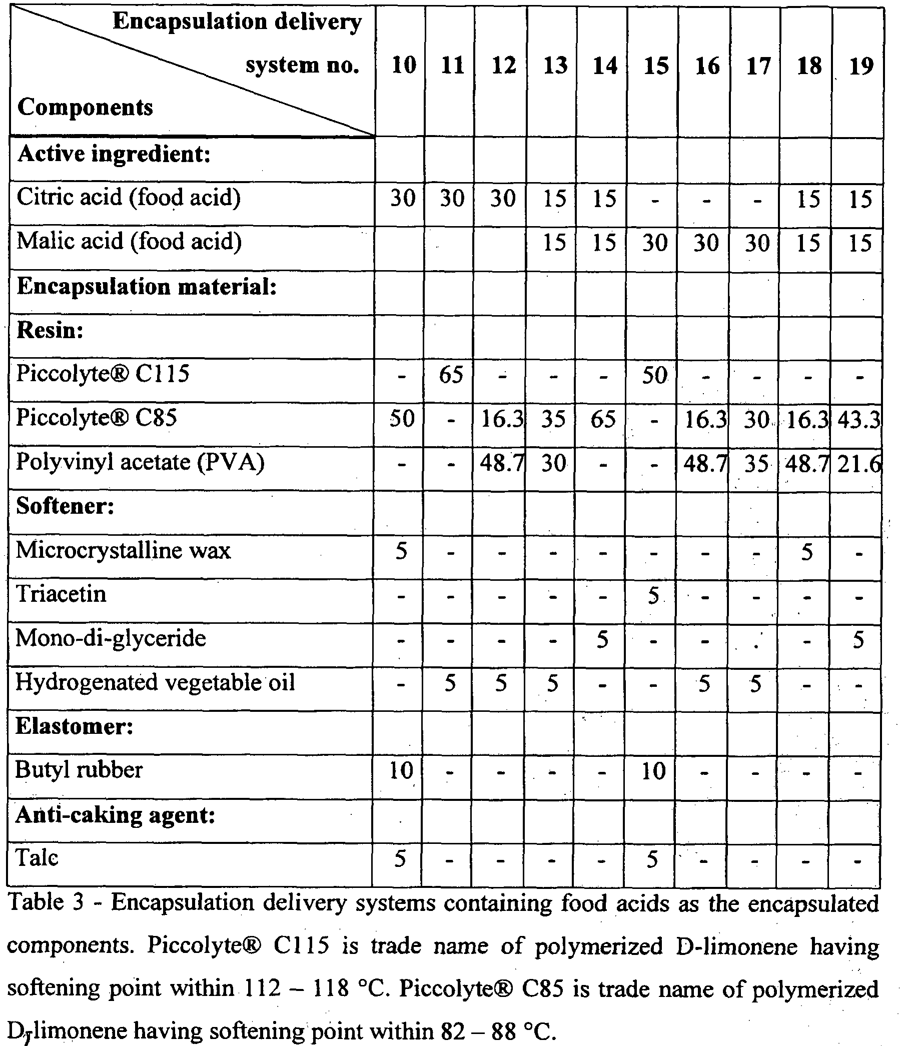

The encapsulation delivery systems no. 10-11, 14-15 are not part of the invention.

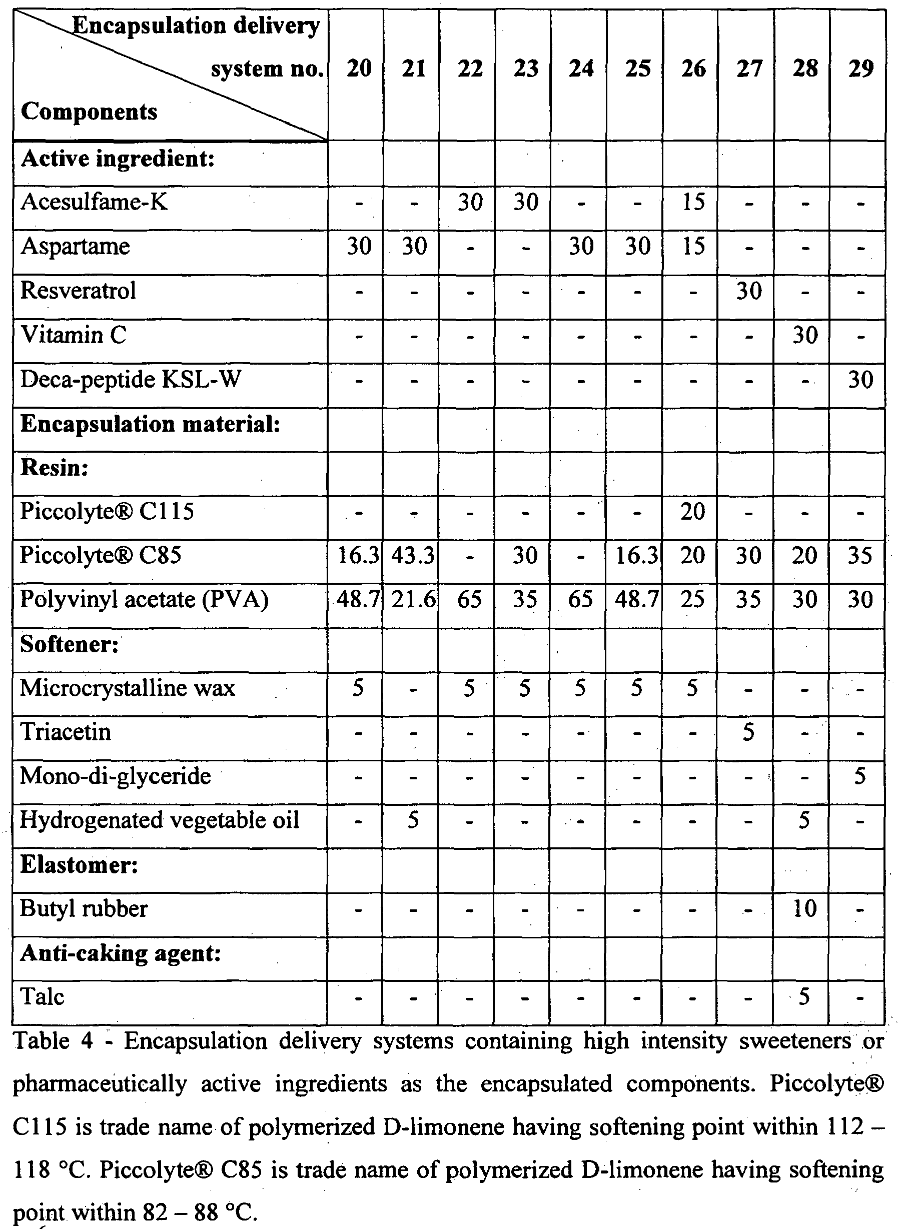

The encapsulation delivery systems no. 22, 24 are not part of the invention.

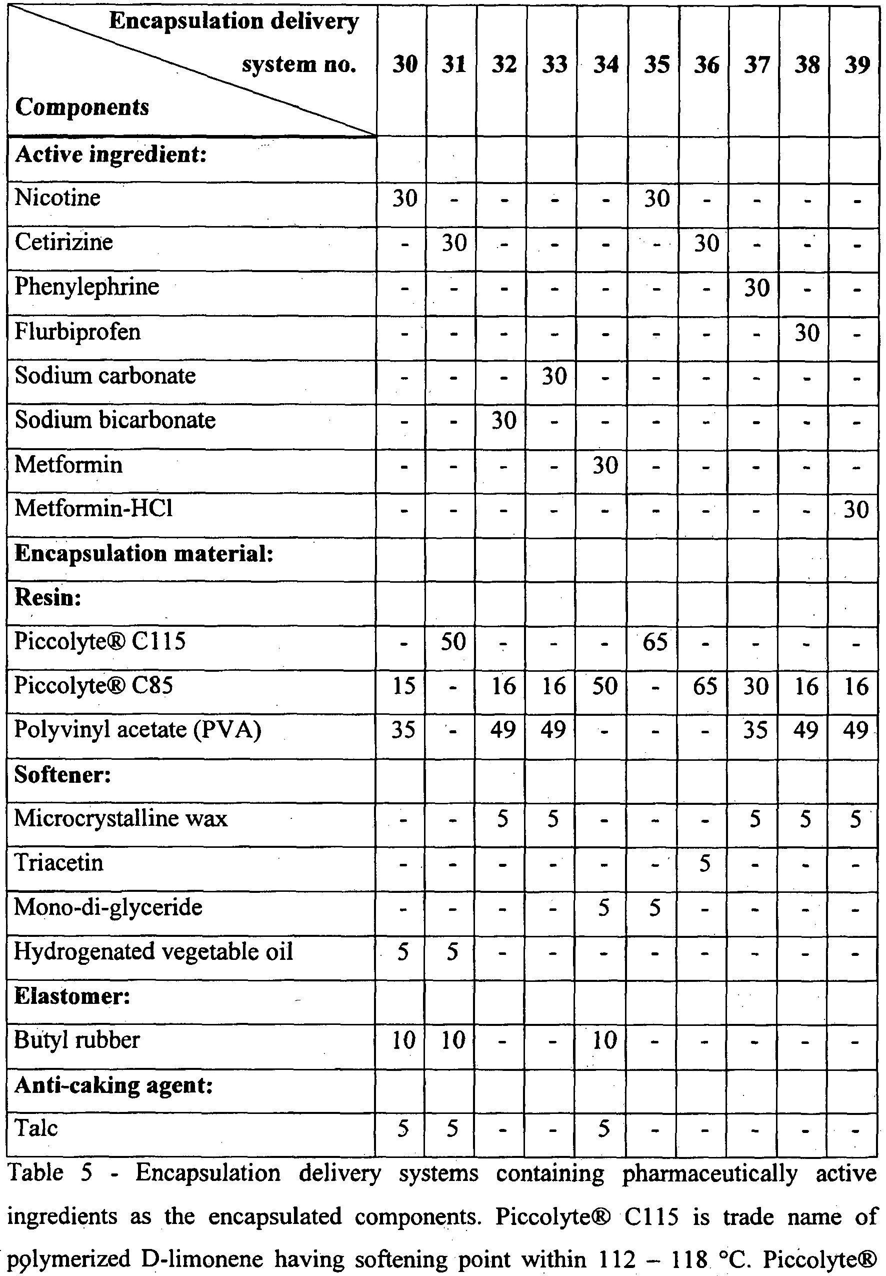

The encapsulation delivery systems no. 31, 34, 35, 36 are not part of the invention.

C85 is trade name of polymerized D-limonene having softening point within 82 - 88 °C.

### EXAMPLE 3

### Compressible chewing gum compositions

Encapsulation delivery system no. 10-39 may be applied in compressible chewing gum composition no. 1010-1039, respectively (encapsulation delivery system no. 10 in compressible chewing gum composition no. 1010 etc). The compressible chewing gum compositions comprising the encapsulation delivery systems no. 10-11, 14-15, 22, 24, 31, 34-36 are not part of the invention.

Compressible chewing gum compositions were prepared with the formulations given in table 6, table 7, and table 8.

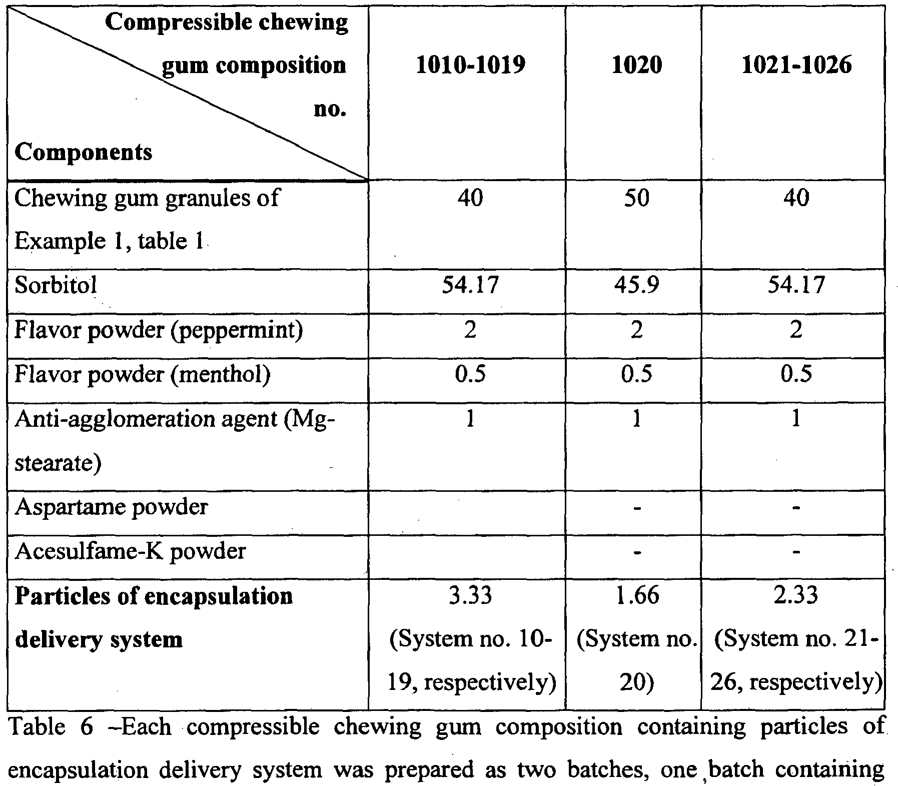

encapsulation delivery system of particle size 106-500 and one batch containing encapsulation delivery system of particle size 500-1000.

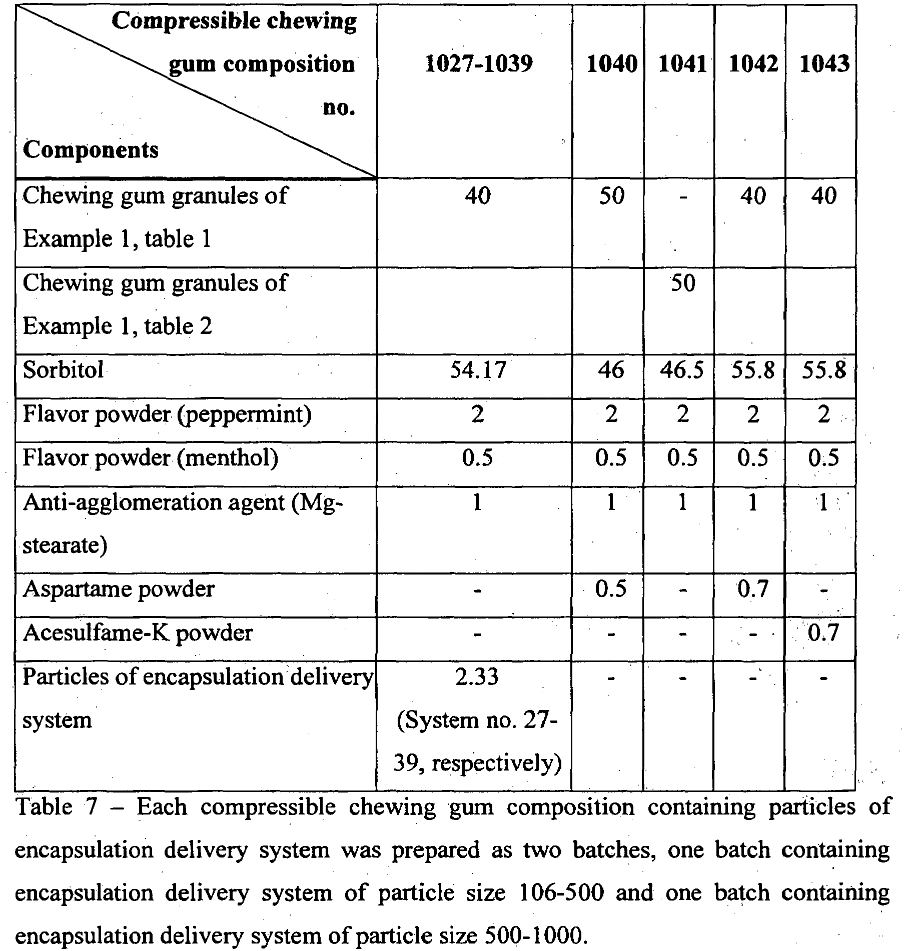

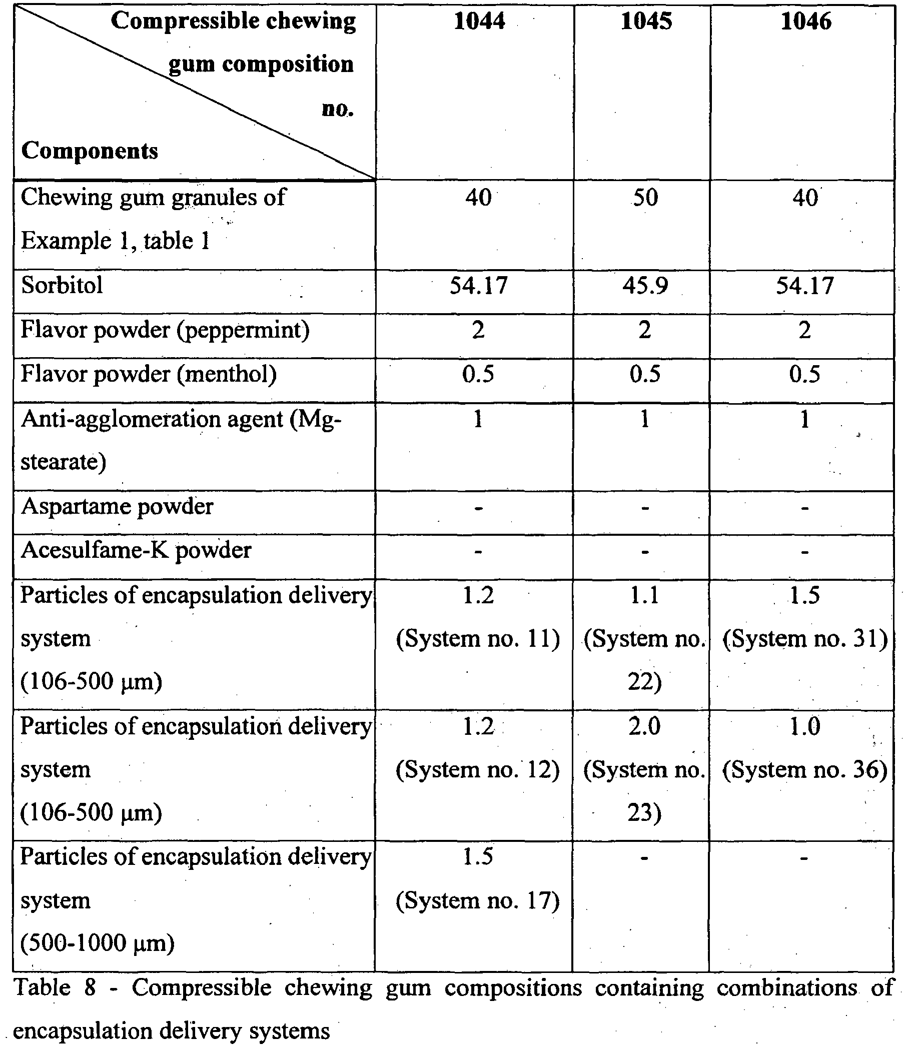

The compressible chewing gum compositions were compressed in a standard tablet pressing machine comprising dosing apparatus (P 3200 C, available from Fette GmbH, Germany) and pressed into compressed tablets at a temperature of below 20°C and a relative humidity of about 50%. The tablets were precompressed and then main compressed to a diameter of 16.0 mm and a center height of 9 mm using a pressing pressure of 33 kN.

### EXAMPLE 4

### Evaluation of encapsulation delivery systems in compressed chewing gum Encapsulation vs. no encapsulation

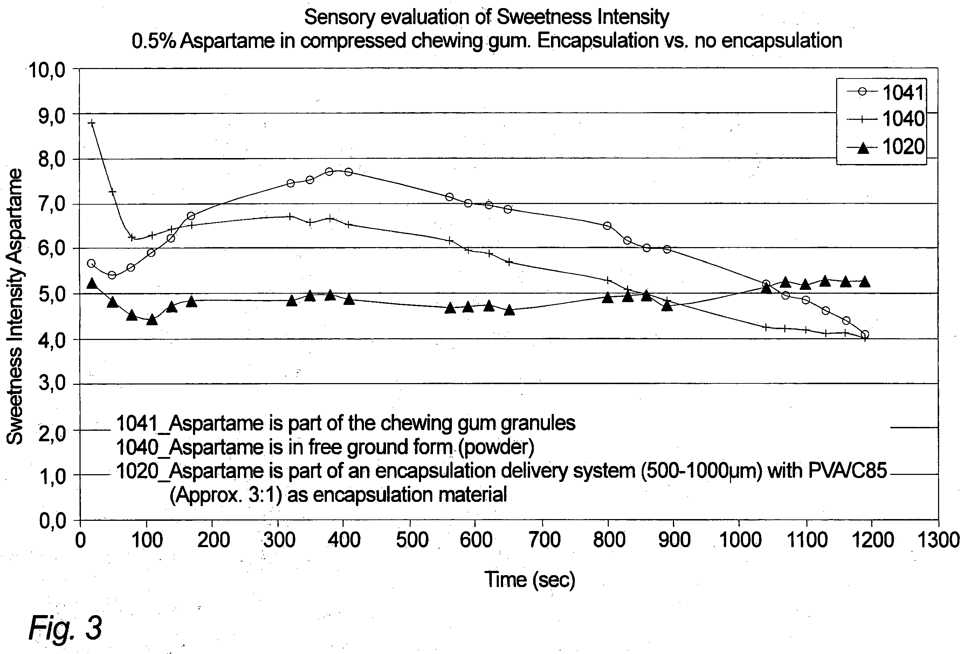

Fig. 3 illustrates results obtained from a taste panel blind testing the sweetness intensity of compressed chewing gum tablets based on compressible chewing gum compositions no. 1020, 1040, and 1041 during a chewing period of 20 minutes.
It is noted that an advantageous effect with regard to release is obtained when the active ingredient, in this case aspartame, is included in an encapsulation delivery system (in comp no. 1020). The intensity of 1020 is almost the same throughout the chewing period, from the first to the last minute. This is very much in contrast both to 1040 and 1041.
In 1040, the intensity is very pronounced in the beginning, as the aspartame added as free powder is released very quickly from the compressed chewing gum. When the intensity falls radically in the first 1 - 1.5 minutes and continues to fall, the experience of chewing the chewing gum is that it loses its taste.
In 1041, the aspartame is included in the chewing gum granules, and the resulting release profile is found as a gradual increase in intensity more or less during the first 5-6 minutes, but then the intensity starts to fall continuously during the rest of the chewing period, and the experience is again that the compressed chewing gum loses its taste.
There is a marked difference between 1020, i.e. the composition comprising the encapsulation delivery system, and the two alternative locations provided in compositions 1040 and 1041.
The encapsulation delivery system comprising polyvinyl acetate and polyterpene resin as encapsulation material has shown to provide an indeed very effective encapsulation of active ingredient, here in the form of aspartame.

### EXAMPLE 5

### Evaluation of encapsulation delivery systems in compressed chewing gum

### Encapsulation in polyvinyl acetate and polyterpene resin

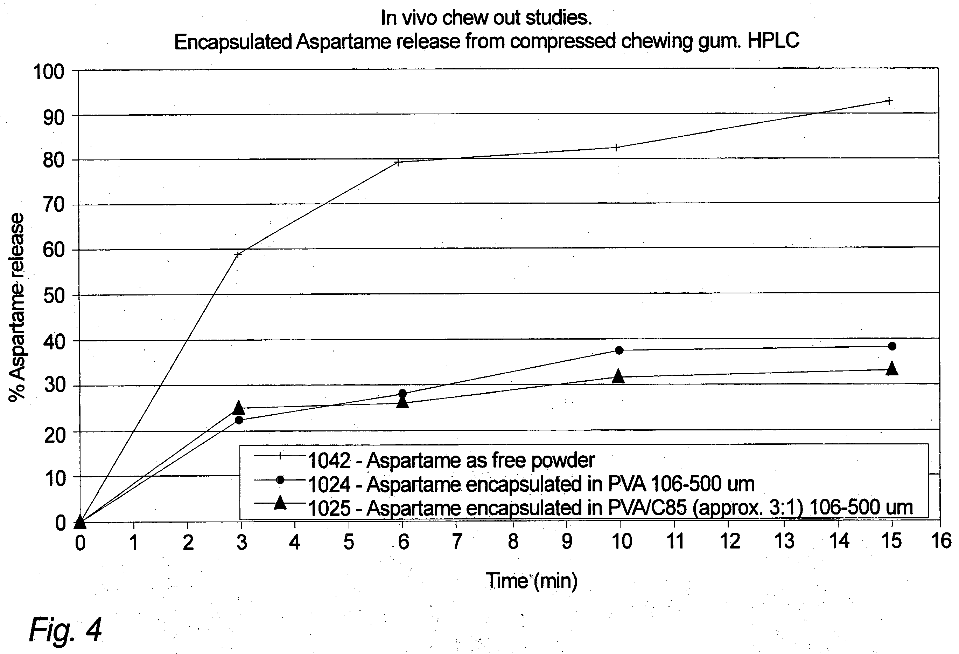

Fig. 4 illustrates % release of aspartame from compressed chewing gum tablets by in vivo chew out studies measured by HPLC. The compressed chewing gum tablets are based on compressible chewing gum compositions no. 1024, 1025, and 1042.

In 1042, active ingredient, here aspartame, is added as free particles (powder) to the chewing gum composition, and it is seen that the release is very fast. Within the first 3 minutes of chewing, 60% of the active ingredient is released from the compressed chewing gum, and within the first 6 minutes, 80% is released.

In contrast, both 1024 and 1025, in which the active ingredient is encapsulated, the release is reduced radically. It appears clearly that encapsulation in PVA:polyterpene resin (in a ratio of approx. 3:1) is as effective as or even more effective than encapsulation in PVA alone.

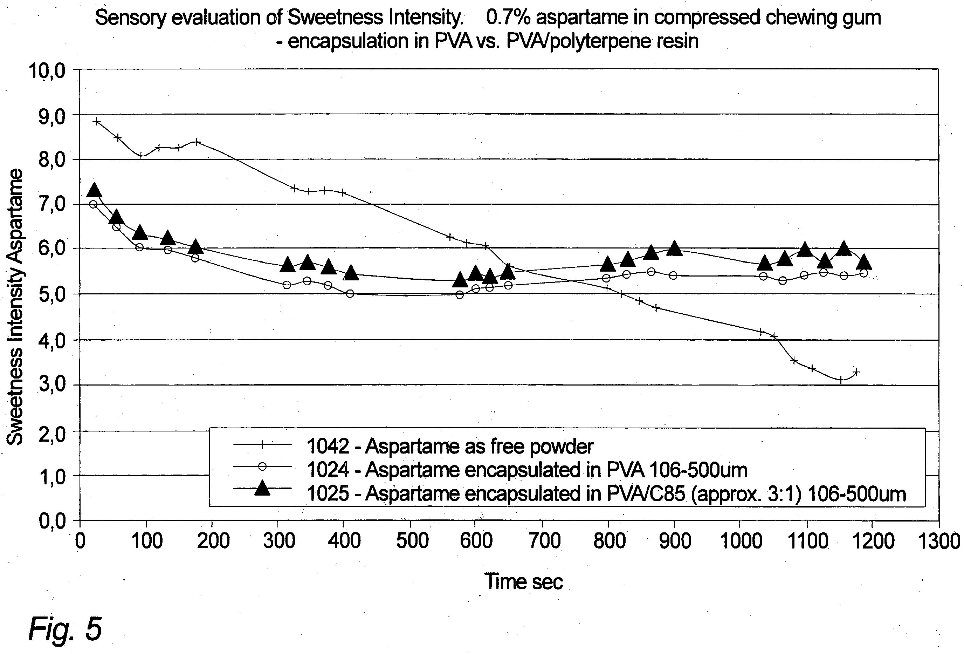

Fig. 5 illustrates results obtained from a taste panel blind testing the sweetness intensity of compressed chewing gum tablets corresponding to those tested by HPLC in fig. 4, i.e. compressed chewing gum based on compressible chewing gum compositions no. 1042, 1024, and 1025. The chewing period is 20 minutes. It is noted that while the intensity of aspartame added as free powder is falling throughout the chewing period, the intensity obtained when aspartame is encapsulated is relatively constant. It appears that as a result of the encapsulation, the intensity is more or less on the same level throughout the chewing period, which means that the experience when chewing the compressed chewing gum is that it continues to maintain its taste. In fact, in this case, the intensity of the compressed chewing gum in which natural resin has been applied (1025) is constantly even higher compared to encapsulation in PVA alone (1024).

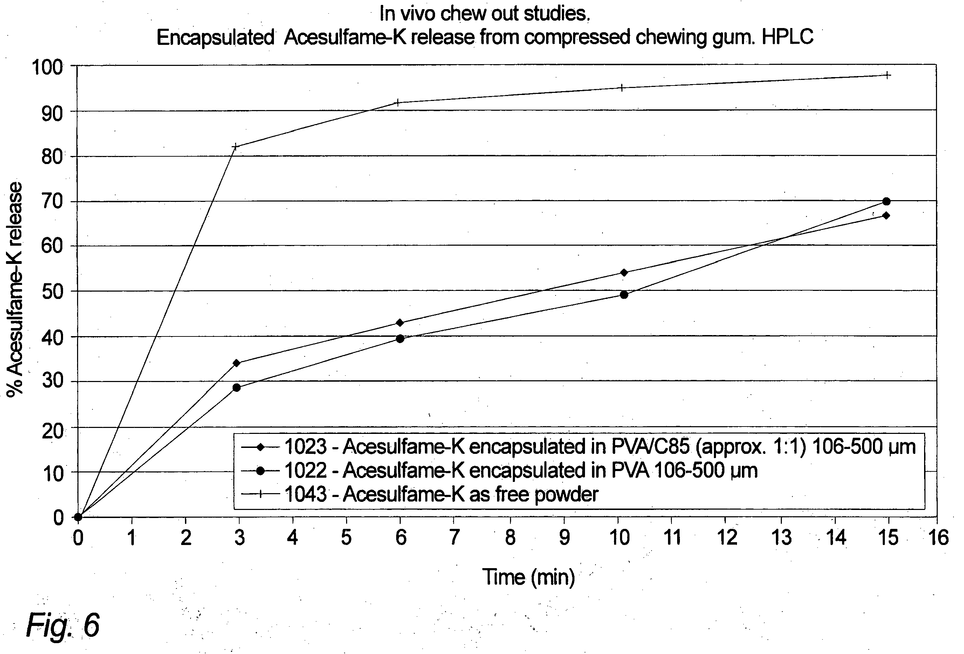

Fig. 6 illustrates % release of acesulfame-K from compressed chewing gum tablets by in vivo chew out studies measured by HPLC. The compressed chewing gum tablets are based on compressible chewing gum compositions no. 1023, 1022, and 1043. It appears clearly that encapsulation in PVA:polyterpene resin (in a ratio of approx. 1:1) is as effective as encapsulation in PVA alone.

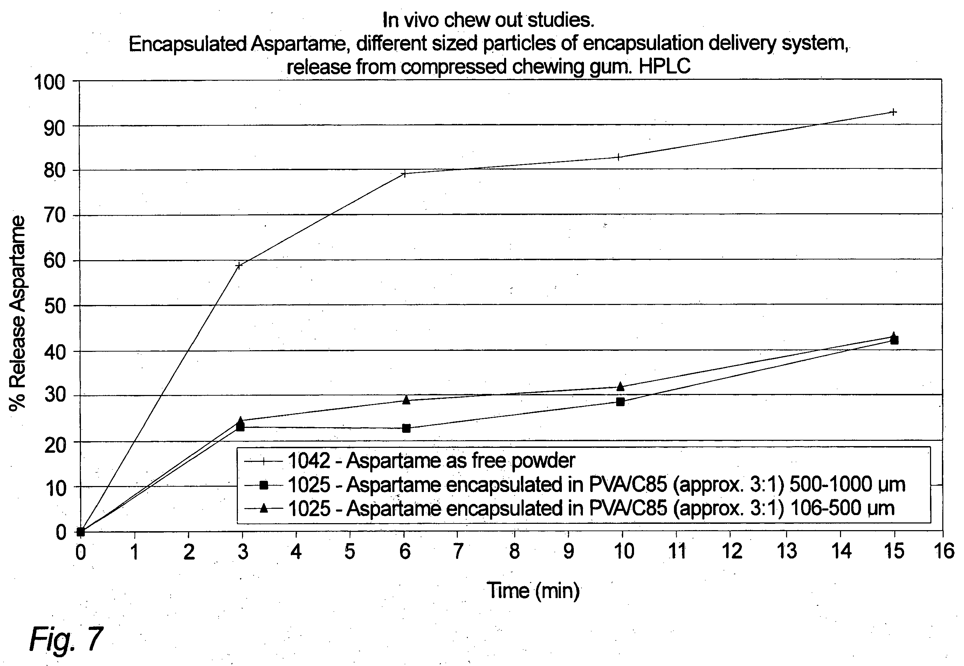

Fig. 7 illustrates % release of aspartame from compressed chewing gum tablets by in vivo chew out studies measured by HPLC. The compressed chewing gum tablets are based on compressible chewing gum compositions no. 1042, in which aspartame is added as free particles (powder) and 1025, in which aspartame is encapsulated in PVA:polyterpene resin (in a ratio of approx. 3:1). Two different particle sizes (106-500 vs. 500-1000) of encapsulation delivery system particles in 1025 are compared, and it appears that the % release during the chewing period is roughly on the same level regardless of the difference in particle size, although there is a tendency that the smaller particles release more than the larger particles.

## Claims

1. Compressible chewing gum composition comprising
i) chewing gum granules containing gum base
ii) particles of one or more encapsulation delivery systems comprising at least one encapsulation material and at least one active ingredient being encapsulated by the encapsulation material,
wherein the encapsulation material comprises at least one natural resin and wherein said encapsulation material further comprises polyvinyl acetate, and
wherein there is a ratio of said natural resin to said polyvinyl acetate in the range of 95:5 to 5:95, preferably in the range of 70:30 to 15:85 such as in the range of 60:40 to 20:80.

2. Compressible chewing gum composition according to claim 1, wherein said at least one natural resin constitutes in the range of 15 % to 95 %, preferably in the range of 25 % to 95 % by weight of said encapsulation material.

3. Compressible chewing gum composition according to claim 1 or 2, wherein at least one of said encapsulation delivery systems comprises a combination of two or more natural resins.

4. Compressible chewing gum composition according to any of the claims 1-3, wherein the compressible chewing gum composition comprises an amount of the one or more encapsulation delivery systems in the range of 0.5 % to 20 % by weight, preferably in the range of 1 % to 10 % by weight, and most preferably in the range of 2 % to 6 % by weight of the composition.

5. Compressible chewing gum composition according to any of the claims 1-4, wherein the size of the particles of said one or more encapsulation delivery systems 150804. Application no. 07766611.3
is in the range of 100 - 3000 µm, preferably in the range of 100 - 2000 µm, preferably 100-1000 such as in the range of 100 - 600 µm.

6. Compressible chewing gum composition according to any of the claims 1-5, wherein said at least one natural resin is selected from the group consisting of polyterpene resins, hydrogenated resins, polymerized resins, or any combination thereof.

7. Compressible chewing gum composition according to any of the claims 1-6, wherein two or more encapsulation delivery systems comprise two or more active ingredients.

8. Compressible chewing gum composition according to any of the claims 1-7, wherein the one or more encapsulation delivery systems comprise at least a first encapsulation delivery system and at least a second encapsulation delivery system comprising different percentages of natural resin.

9. Compressible chewing gum composition according to any of the claims 1-8, wherein the one or more encapsulation delivery systems comprise at least a first encapsulation delivery system and at least a second encapsulation delivery system comprising different encapsulation material.

10. Compressible chewing gum composition according to any of the claims 1-9, wherein the one or more encapsulation delivery systems comprise at least a first encapsulation delivery system comprising at least a first active ingredient and at least a second encapsulation delivery system comprising at least a second active ingredient.

11. Compressible chewing gum composition according to any of the claims 1-10, wherein the active ingredient is distributed in the encapsulation delivery system as 150804. Application no. 07766611.3
particles with particle sizes in the range of 0.1-100 µm, preferably in the range of 1-50 µm.

12. Compressible chewing gum composition according to any of the claims 1-11, wherein the chewing gum granules are free of active ingredient.

13. Compressed chewing gum tablet comprising a compressible chewing gum composition according to any of the claims 1-12.

14. Method of providing a compressed chewing gum tablet from the compressible chewing gum composition according to any of the claims 1-12 comprising the step of compressing the compressible chewing gum composition in a tabletting press.

## Patentansprüche

1. Pressbare Kaugummimischung, Folgendes umfassend:
i) Kaugummikörnchen, die Kaugummi-Grundmasse enthalten,
ii) Teilchen von einem oder mehreren Verkapselungs-Verabreichungssystemen, die wenigstens ein Verkapselungsmaterial umfassen sowie wenigstens einen Wirkstoff, der vom Verkapselungsmaterial verkapselt wird,
wobei das Verkapselungsmaterial wenigstens ein Naturharz umfasst und wobei das Verkapselungsmaterial außerdem Polyvinylacetat umfasst, und
wobei ein Verhältnis des Naturharzes zum Polyvinylacetat im Bereich von 95:5 bis 5:95, vorzugsweise im Bereich von 70:30 bis 15:85, wie etwa im Bereich von 60:40 bis 20:80, liegt.

2. Pressbare Kaugummimischung nach Anspruch 1, wobei das wenigstens eine Naturharz im Bereich von 15 % bis 95 % und vorzugsweise im Bereich 25 % bis 95 % nach Gewicht vom Verkapselungsmaterial ausmacht.

3. Pressbare Kaugummimischung nach Anspruch 1 oder 2, wobei wenigstens eines der Verkapselungs-Verabreichungssysteme eine Kombination von zwei oder mehr Naturharzen umfasst.

4. Pressbare Kaugummimischung nach einem der Ansprüche 1 bis 3, wobei die pressbare Kaugummimischung einen Anteil des einen oder der mehreren Verkapselungs-Verabreichungssysteme im Bereich von 0,5 % bis 20 % nach Gewicht, vorzugsweise im Bereich von 1 % bis 10 % nach Gewicht und am bevorzugtesten im Bereich von 2 % bis 6 % nach Gewicht an der Mischung umfasst.

5. Pressbare Kaugummimischung nach einem der Ansprüche 1 bis 4, wobei die Größe der Teilchen des einen oder der mehreren Verkapselungs-Verabreichungssysteme im Bereich von 100 bis 3000 µm, vorzugsweise im Bereich von 100 bis 2000 µm, vorzugsweise 100 bis 1000, wie etwa im Bereich von 100 bis 600 µm, liegt.

6. Pressbare Kaugummimischung nach einem der Ansprüche 1 bis 5, wobei das wenigstens eine Naturharz aus der Gruppe gewählt ist, die aus Folgendem besteht: Polyterpen-Harze, hydrierte Harze, polymerisierte Harze oder eine beliebige Kombination von diesen.

7. Pressbare Kaugummimischung nach einem der Ansprüche 1 bis 6, wobei zwei oder mehr Verkapselungs-Verabreichungssysteme zwei oder mehr Wirkstoffe umfassen.

8. Pressbare Kaugummimischung nach einem der Ansprüche 1 bis 7, wobei das eine oder die mehren Verkapselungs-Verabreichungssysteme wenigstens ein erstes Verkapselungs-Verabreichungssystem und wenigstens ein zweites Verkapselungs-Verabreichungssystem umfassen, die unterschiedliche Anteile an Naturharz umfassen.

9. Pressbare Kaugummimischung nach einem der Ansprüche 1 bis 8, wobei das eine oder die mehren Verkapselungs-Verabreichungssysteme wenigstens ein erstes Verkapselungs-Verabreichungssystem und wenigstens ein zweites Verkapselungs-Verabreichungssystem umfassen, die ein unterschiedliches Verkapselungsmaterial umfassen.

10. Pressbare Kaugummimischung nach einem der Ansprüche 1 bis 9, wobei das eine oder die mehren Verkapselungs-Verabreichungssysteme Folgendes umfassen: wenigstens ein erstes Verkapselungs-Verabreichungssystem, das wenigstens einen ersten Wirkstoff umfasst, und wenigstens ein zweites Verkapselungs-Verabreichungssystem, das wenigstens einen zweiten Wirkstoff umfasst.

11. Pressbare Kaugummimischung nach einem der Ansprüche 1 bis 10, wobei der Wirkstoff im Verkapselungs-Verabreichungssystem in Form von Teilchen verteilt ist mit einer Teilchengröße im Bereich von 0,1 bis 100 µm und vorzugsweise im Bereich von 1 bis 50 µm.

12. Pressbare Kaugummimischung nach einem der Ansprüche 1 bis 11, wobei die Kaugummikörnchen wirkstofffrei sind.

13. Gepresste Kaugummitablette, die eine pressbare Kaugummimischung nach einem der Ansprüche 1 bis 12 umfasst.

14. Verfahren zum Bereitstellen einer gepressten Kaugummitablette aus der pressbaren Kaugummimischung nach einem der Ansprüche 1 bis 12, das den folgenden Schritt umfasst: Pressen der pressbaren Kaugummimischung in einer Tablettenpresse.

## Revendications

1. Composition de gomme à mâcher compressible comprenant
i) des granules de gomme à mâcher contenant une base de gomme
ii) des particules d'un ou de plusieurs systèmes de délivrance par encapsulation comprenant au moins un matériau d'encapsulation et au moins un ingrédient actif encapsulé par le matériau d'encapsulation,
dans laquelle le matériau d'encapsulation comprend au moins une résine naturelle et dans laquelle ledit matériau d'encapsulation comprend en outre un poly(acétate de vinyle), et
dans laquelle il existe un rapport entre ladite résine naturelle et ledit poly(acétate de vinyle) situé dans la plage allant de 95/5 à 5/95, de préférence dans la plage allant de 70/30 à 15/85, comme dans la plage allant de 60/40 à 20/80.

2. Composition de gomme à mâcher compressible selon la revendication 1, dans laquelle ladite au moins une résine naturelle constitue de 15 % à 95 %, de préférence de 25 % à 95 % en poids dudit matériau d'encapsulation.

3. Composition de gomme à mâcher compressible selon la revendication 1 ou 2, dans laquelle au moins un desdits systèmes de délivrance par encapsulation comprend une combinaison de deux ou plus de deux résines naturelles.

4. Composition de gomme à mâcher compressible selon l'une quelconque des revendications 1 à 3, dans laquelle la composition de gomme à mâcher compressible comprend une quantité du ou des systèmes de délivrance par encapsulation située dans la plage allant de 0,5 % à 20 % en poids, de préférence dans la plage allant de 1 % à 10 % en poids, et de manière préférée entre toutes dans la plage allant de 2 % à 6 % en poids de la composition.

5. Composition de gomme à mâcher compressible selon l'une quelconque des revendications 1 à 4, dans laquelle la taille des particules du ou des systèmes de délivrance par encapsulation est située dans la plage allant de 100 µm à 3000 µm, de préférence dans la plage allant de 100 µm à 2000 µm, de préférence de 100 µm à 1000 µm, comme dans la plage allant de 100 µm à 600 µm.

6. Composition de gomme à mâcher compressible selon l'une quelconque des revendications 1 à 5, dans laquelle ladite au moins une résine naturelle est choisie dans le groupe constitué des résines de polyterpène, des résines hydrogénées, des résines polymérisées ou de l'une quelconque de leurs combinaisons.

7. Composition de gomme à mâcher compressible selon l'une quelconque des revendications 1 à 6, dans laquelle deux ou plus de deux systèmes de délivrance par encapsulation comprennent deux ou plus de deux ingrédients actifs.

8. Composition de gomme à mâcher compressible selon l'une quelconque des revendications 1 à 7, dans laquelle le ou les systèmes de délivrance par encapsulation comprennent au moins un premier système de délivrance par encapsulation et au moins un deuxième système de délivrance par encapsulation comprenant différents pourcentages de résine naturelle.

9. Composition de gomme à mâcher compressible selon l'une quelconque des revendications 1 à 8, dans laquelle le ou les systèmes de délivrance par encapsulation comprennent au moins un premier système de délivrance par encapsulation et au moins un deuxième système de délivrance par encapsulation comprenant un matériau d'encapsulation différent.

10. Composition de gomme à mâcher compressible selon l'une quelconque des revendications 1 à 9, dans laquelle le ou les systèmes de délivrance par encapsulation comprennent au moins un premier système de délivrance par encapsulation comprenant au moins un premier ingrédient actif et au moins un deuxième système de délivrance par encapsulation comprenant au moins un deuxième ingrédient actif.

11. Composition de gomme à mâcher compressible selon l'une quelconque des revendications 1 à 10, dans laquelle l'ingrédient actif est réparti dans le système de délivrance par encapsulation sous la forme de particules ayant des tailles de particule situées dans la plage allant de 0,1 µm à 100 µm, de préférence dans la plage allant de 1 µm à 50 µm.

12. Composition de gomme à mâcher compressible selon l'une quelconque des revendications 1 à 11, dans laquelle les granules de gomme à mâcher sont exempts d'ingrédient actif.

13. Tablette de gomme à mâcher compressée comprenant une composition de gomme à mâcher compressible selon l'une quelconque des revendications 1 à 12.

14. Procédé d'obtention d'une tablette de gomme à mâcher compressée à partir de la composition de gomme à mâcher compressible selon l'une quelconque des revendications 1 à 12, comprenant l'étape de compression de la composition de gomme à mâcher compressible dans une presse à tablettes.
